# EUROPEAN PATENT APPLICATION

(11) **EP 4 799 614 A1**
(43) Date of publication of application: **02.09.2026**
(21) Application number: 24882819.6
(22) Date of filing: 23.10.2024
(51) Int. Cl.: A61K 9/51, A61K 48/00, A61K 47/54, A61K 47/69, A61K 47/18, A61K 47/14

(54) **NANOPARTICLE COMPOSITION FOR DRUG DELIVERY**

(30) Priority: 24.10.2023 KR 20230143277
(71) Applicant: SAMYANG BIOPHARM CORPORATION, Seongnam-si Gyeonggi-do 13488 (KR)
(72) Inventor: KIM, Sol, Seongnam-si Gyeonggi-do 13488 (KR); YUM, Kyu Hee, Seoul 06346 (KR); CHOI, Ji Hye, Seoul 05615 (KR); PARK, Jong Min, Hwaseong-si Gyeonggi-do 18484 (KR); LEE, So Jin, Seongnam-si Gyeonggi-do 13479 (KR)
(74) Representative: Gleiss Große Schrell und Partner mbB
(86) International application number: PCT/KR2024/016197
(87) International publication number: WO 2025/089792

(57) **Abstract**

The present invention relates to a composition for drug delivery and a preparation method therefor and, more specifically, to: a composition for drug delivery which is formed such that a drug is encapsulated inside a nanoparticle structure formed by a polymer and a cationic lipid having a specific structure; and a preparation method therefor.

## Description

### TECHNICAL FIELD

The present invention relates to a nanoparticle composition for drug delivery and a method for preparing the same, and more specifically, a composition for drug delivery which is in a form where a drug is encapsulated within a nanoparticle structure formed by a polymer and a cationic lipid having a specific structure, and a method for preparing the same.

### BACKGROUND ART

In therapies using anionic drugs including nucleic acid, technologies for safe and efficient drug delivery have been researched for a long time, and various carriers and techniques for delivery have been developed. Carriers are mainly divided into viral carriers utilizing adenovirus, retrovirus or the like, and non-viral carriers utilizing cationic lipid, cationic polymer or the like. Viral carriers are known as being exposed to risks such as non-specific immune response, etc. Thus, recent researches proceed in the direction to improve such disadvantages by using non-viral carriers. Although non-viral carriers are less efficient in comparison with viral carriers, they have advantages of fewer side effects in terms of *in vivo* safety.

The representative non-viral carriers for delivering nucleic acid material are a complex of cationic lipid and nucleic acid (lipoplex) and a complex of polycationic polymer and nucleic acid (polyplex). Such a cationic lipid or polycationic polymer stabilizes anionic drug by forming a complex through electrostatic interaction with the anionic drug and increases intracellular delivery, and for these reasons, various researches thereof have been conducted. However, when they are administered intravenously in an amount required to obtain a sufficient effect, severe toxicity is caused, although less toxic than viral carriers, resulting in that they are unsuitable for use in pharmaceutical products. Accordingly, there is a need to develop anionic drug delivery technology that is stable *in vivo* and capable of intracellular delivery to obtain sufficient effects, while reducing toxicity by minimizing the use of cationic polymer or cationic lipid that may cause toxicity.

Various anionic drug delivery compositions and preparation methods thereof have been disclosed, wherein a complex is formed by electrostatic interaction between nucleic acid and cationic lipid, and said complex is encapsulated within a nanoparticle structure of amphiphilic block copolymer. For example, Korean Laid-open Patent Publication No. 10-2017-0032858 discloses a composition for delivering an anionic drug, comprising the anionic drug as an active ingredient; a cationic compound; an amphiphilic block copolymer; and a salt of polylactic acid, wherein the anionic drug forms a complex with the cationic compound by electrostatic interaction, and the formed complex is encapsulated within the nanoparticle structure formed by the amphiphilic block copolymer and the salt of polylactic acid, and a method for preparing the same. However, including those disclosed in the above patent publication, the existing nanoparticle drug delivery systems still lack the efficiency to deliver drugs such as nucleic acid, polypeptide, or virus (especially mRNA) into the body.

### CONTENTS OF THE INVENTION

### PROBLEMS TO BE SOLVED

The purpose of the present invention is to provide a composition for drug delivery having significantly improved *in vivo* delivery efficiency of drugs such as nucleic acid, polypeptide, or virus (especially mRNA), as compared with previously known nanoparticle drug delivery systems, and a method for preparing the same.

### TECHNICAL MEANS

The first aspect of the present invention provides a composition for drug delivery comprising: effective ingredient selected from nucleic acid, polypeptide, virus or combination thereof; a lipid having a structure represented by the following formula 1; and lipid-polymer, amphiphilic block copolymer, or a combination thereof: 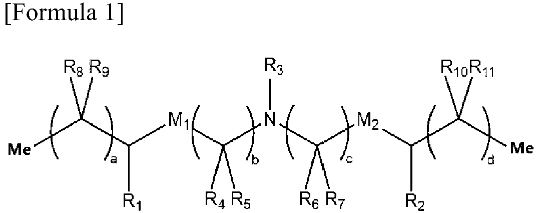 wherein, in the above formula 1,
each of M₁ and M₂ is independently a divalent linker group,
each of R₁ and R₂ is independently a substituted or unsubstituted carbocyclic group or heterocyclic group,
R₃ is hydrogen atom, or a substituted or unsubstituted organic group optionally comprising one or more heteroatoms,
each of R₄ to R₁₁ is independently hydrogen atom, or a substituted or unsubstituted, saturated or unsaturated hydrocarbon group,
Me is methyl group, and
each of a, b, c and d is independently an integer of from 1 to 20.

The second aspect of the present invention provides a method for preparing a composition for drug delivery, comprising the steps of: (a) preparing a solution in which a lipid represented by the above formula 1; and lipid-polymer, amphiphilic block copolymer, or a mixture thereof; are dissolved in a water-miscible organic solvent; and (b) to the solution prepared in step (a), adding effective ingredient selected from nucleic acid, polypeptide, virus, or combination thereof, and mixing them.

### EFFECT OF THE INVENTION

The composition for drug delivery according to the present invention can significantly improve *in vivo* delivery efficiency of drugs such as nucleic acid, polypeptide, or virus (especially mRNA), as compared with previously known nanoparticle drug delivery systems.

### CONCRETE MODE FOR CARRYING OUT THE INVENTION

The present invention will be explained in detail below.

### Effective ingredient

The effective ingredient comprised in the composition for drug delivery of the present invention is selected from nucleic acid, polypeptide, virus, or combination thereof.

The "nucleic acid" may be, for example, DNA, RNA, siRNA, shRNA, miRNA, mRNA, aptamer, antisense oligonucleotide, or a combination thereof, but it is not limited thereto.

The "polypeptide" may mean a protein having activity in the body such as antibody or fragment thereof, cytokine, hormone or analog thereof, or a protein that can be recognized as antigen through a series of processes in the body, including polypeptide sequence of antigen, analog or precursor thereof.

The "virus" may be an oncolytic virus and, for example, may be one or more selected from the group consisting of adenovirus, AAV, vaccinia virus, herpes simplex virus (HSV), and vesicular stomatitis virus (VSV). In an embodiment, the oncolytic virus is an adenovirus. The adenovirus used in an embodiment of the present invention contains a luciferase gene, which can be confirmed through imaging.

The virus can express various types of therapeutic genes within the body of an individual and is not limited to specific molecular weight, protein, bioactivity or therapeutic field. The prophylactic virus can induce immunity within the body of an individual against a target disease. A composition containing a prophylactic virus to disease has the advantage of reducing immunity induction by the virus itself, capability of designating or expanding target cells, and reducing the hyperimmune response to the virus upon re-administration, thereby enabling effective effects to be obtained through multiple inoculations.

In an embodiment, the effective ingredient is mRNA (messenger RNA).

The mRNA may be changed chemically in its backbone, sugar or base modified or modified at the end, for purposes such as increasing blood stability or weakening the immune response, etc.

Specifically, some of the phosphodiester bonds of mRNA may be replaced with phosphorothioate or boranophosphate bond, or one or more modified nucleotides may be included in which various functional groups such as methyl group, methoxyethyl group, and fluorine are introduced at the 2'-OH position of some ribose bases.

In addition, one or more ends of the mRNA may be modified with one or more selected from the group consisting of cholesterol, tocopherol and fatty acids having 10 to 24 carbon atoms. The cholesterol, tocopherol and fatty acids having 10 to 24 carbon atoms include each analogue, derivative and metabolite of the cholesterol, tocopherol and fatty acids.

In an embodiment, the amount of the effective ingredient may be, based on the dry weight of the total composition, 0.05 wt% or more, 0.1 wt% or more, 0.2 wt% or more, 0.3 wt% or more, 0.4 wt% or more, or 0.5 wt% or more, and it may also be 10 wt% or less, 9 wt% or less, 8 wt% or less, 7 wt% or less, 6 wt% or less, 5 wt% or less, 4 wt% or less, or 3 wt% or less. If the amount of the effective ingredient is too less, the amount of delivery carrier becomes too much as compared with the drug, and thus there may be a side effect due to the delivery carrier. To the contrary, if the amount of the effective ingredient is too much, the amount of drug not encapsulated in nanoparticles becomes too much, and thus the efficiency decreases.

### Lipid

The lipid comprised in the nanoparticle composition of the present invention is has a structure represented by the following formula 1: 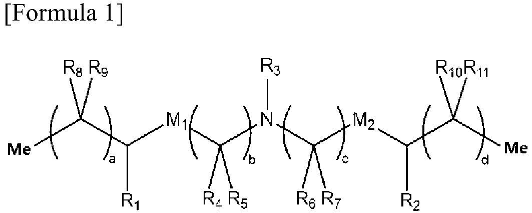 wherein, in the above formula 1,
each of M₁ and M₂ is independently a divalent linker group,
each of R₁ and R₂ is independently a substituted or unsubstituted carbocyclic group or heterocyclic group,
R₃ is hydrogen atom, or a substituted or unsubstituted organic group optionally comprising one or more heteroatoms,
each of R₄ to R₁₁ is independently hydrogen atom, or a substituted or unsubstituted, saturated or unsaturated hydrocarbon group,
Me is methyl group, and
each of a, b, c and d is independently an integer of from 1 to 20.

The scope of the lipid comprised in the drug delivery composition of the present invention includes not only those having the structure of the above formula 1 but also cationic forms thereof.

As used herein, the expression "substituted or unsubstituted" for any group means that, unless specified otherwise, the group is not substituted, or is substituted with hydroxy group or C₁₋₆ alkyl group.

According to an embodiment of the present invention, in the above formula 1, each of M₁ and M₂ may be independently selected from the group consisting of -C(O)O-, -OC(O)-, -OC(O)-M'-C(O)O-, -C(O)N(R')-, -N(R')C(O)-, -C(O)-, -C(S)-, -C(S)S-, -SC(S)-, -CH(OH)-, - P(O)(OR')O-, -S(O)₂-, -S-S-, arylene (more concretely C₆₋₂₀ arylene, still more concretely C₆₋₁₀ arylene), and heteroarylene (more concretely C₃₋₂₀ heteroarylene, still more concretely C₃₋₁₀ heteroarylene, having one or more (e.g., 1 to 3) heteroatoms selected from N, O and S), wherein M' may be a direct bond, C₁₋₁₃ alkylene (more concretely C₁₋₆ alkylene) or C₂₋₁₃ alkenylene (more concretely C₂₋₆ alkenylene), and each R' may be independently selected from the group consisting of hydrogen atom, C₁₋₁₈ alkyl (more concretely C₁₋₁₀ alkyl, still more concretely C₁₋₆ alkyl) and C₂₋₁₈ alkenyl (more concretely C₂₋₁₀ alkenyl, still more concretely C₂₋₆ alkenyl).

According to an embodiment of the present invention, in the above formula 1, each of R₁ and R₂ may be independently selected from the group consisting of substituted or unsubstituted C₃₋₂₀ cycloalkyl (more concretely C₃₋₁₀ cycloalkyl, still more concretely C₃₋₆ cycloalkyl), substituted or unsubstituted C₃₋₂₀ cycloalkenyl (more concretely C₃₋₁₀ cycloalkenyl, still more concretely C₃₋₆ cycloalkenyl), substituted or unsubstituted C₆₋₂₀ aryl (more concretely C₆₋₁₀ aryl, still more concretely C₆ aryl), substituted or unsubstituted C₃₋₂₀ heterocycloalkyl (more concretely C₃₋₁₀ heterocycloalkyl, still more concretely C₃₋₆ heterocycloalkyl), substituted or unsubstituted C₃₋₂₀ heterocycloalkenyl (more concretely C₃₋₁₀ heterocycloalkenyl, still more concretely C₃₋₆ heterocycloalkenyl), and substituted or unsubstituted C₃₋₂₀ heteroaryl (more concretely C₃₋₁₀ heteroaryl, still more concretely C₃₋₆ heteroaryl), wherein each of the heterocycloalkyl, heterocycloalkenyl and heteroaryl may independently have one or more (e.g., 1 to 3) heteroatoms selected from N, O and S.

According to an embodiment of the present invention, in the above formula 1, R₃ may be selected from the group consisting of hydrogen atom, substituted or unsubstituted C₁₋₆ alkyl, substituted or unsubstituted C₃₋₆ carbocyclic group, -(CH₂)ₙQ, - (CH₂)ₙCHQR, -CHQR and - CQ(R)₂, wherein each R may be independently selected from the group consisting of hydrogen atom, C₁₋₃ alkyl and C₂₋₃ alkenyl; Q may be selected from the group consisting of carbocyclic group, heterocyclic group, -OR, -O(CH₂)ₙN(R)₂, -C(O)OR, -OC(O)R, -CX₃, -CX₂H, -CXH₂, -CN, -N(R)₂, -C(O)N(R)₂, -N(R)C(O)R, -N(R)S(O)₂R, -N(R)C(O)N(R)₂, -N(R)C(S)N(R)₂, -N(R)R₁₂, N(R)S(O)2R₁₂, -O(CH₂)ₙOR, -N(R)C(=NR₁₃)N(R)₂, -N(R)C(=CHR₁₃)N(R)₂, -OC(O)N(R)₂, - N(R)C(O)OR, -N(OR)C(O)R, -N(OR)S(O)₂R, -N(OR)C(O)OR, -N(OR)C(O)N(R)₂, - N(OR)C(S)N(R)₂, -N(OR)C(=NR₁₃)N(R)₂, -N(OR)C(=CHR₁₃)N(R)₂, -C(=NR₁₃)N(R)₂, - C(=NR₁₃)R, -C(O)N(R)OR and -C(R)N(R)₂C(O)OR, wherein each n is independently an integer of from 1 to 5; R₁₂ is selected from the group consisting of C₃₋₆ carbocyclic group and heterocyclic group; R₁₃ is selected from the group consisting of H, CN, NO₂, C₁₋₆ alkyl, -OR, -S(O)₂R, - S(O)₂N(R)₂, C₂₋₆ alkenyl, C₃₋₆ carbocyclic group and heterocyclic group; each R is independently selected from the group consisting of hydrogen atom, C₁₋₃ alkyl and C₂₋₃ alkenyl; each X is independently selected from the group consisting of F, CI, Br and I, provided that when R₃ is - (CH₂)ₙQ, -(CH₂)ₙCHQR, -CHQR or -CQ(R)₂, (i) if n is 1, 2, 3, 4, or 5, then Q is not -N(R)₂, or (ii) if n is 1 or 2, Q is not 5-, 6- or 7-membered heterocycloalkyl.

According to an embodiment of the present invention, in the above formula 1, each of R₄ to R₁₁ may be independently selected from the group consisting of hydrogen atom, C₁₋₃ alkyl and C₂₋₃ alkenyl.

According to an embodiment of the present invention, in the above formula 1, each of a, b, c and d may be independently an integer of from 1 to 15.

Still more concretely, in the above formula 1, each of M₁ and M₂ may be independently - C(O)O- or -OC(O)-.

Still more concretely, in the above formula 1, each of R₁ and R₂ may be independently substituted or unsubstituted C₃₋₆ cycloalkyl.

Still more concretely, in the above formula 1, R₃ may be hydrogen atom, or substituted or unsubstituted C₁₋₃ alkyl, and even more concretely, unsubstituted C₁₋₃ alkyl or hydroxy-substituted C₁₋₃ alkyl.

Still more concretely, in the above formula 1, R₄ to R₁₁ may be hydrogen atom.

Still more concretely, in the above formula 1, each of a, b, c and d may be independently an integer of from 3 to 11, and even more concretely, an integer of from 5 to 9.

Even more concretely, the lipid may be one having a structure selected from the following formulas A to O:

| Formula | Structure |
|---|---|
| A | 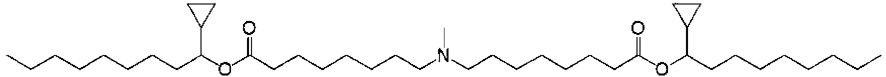 |
| B | 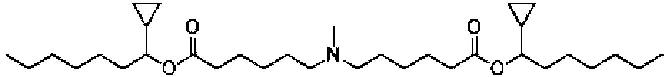 |
| C | 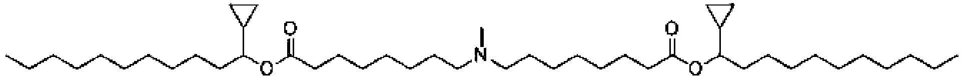 |
| D | 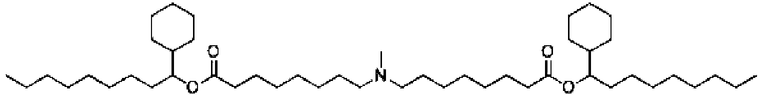 |
| E | 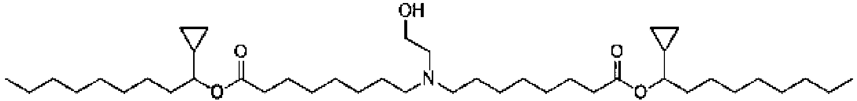 |
| F | 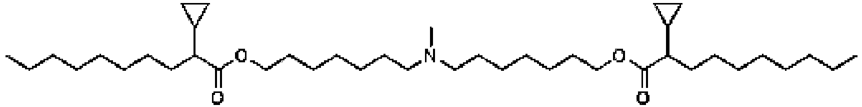 |
| G | 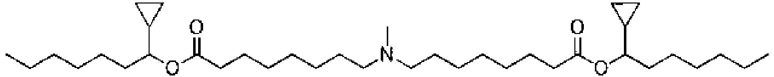 |
| H | 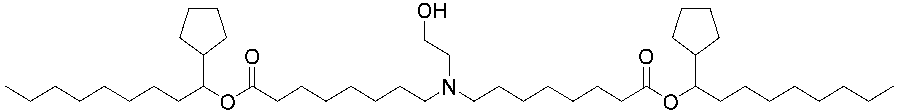 |
| I | 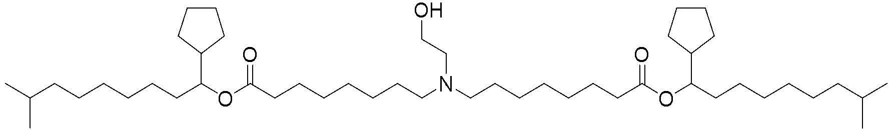 |
| J | 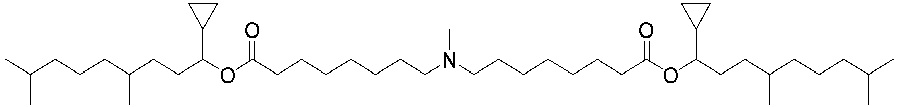 |
| K | 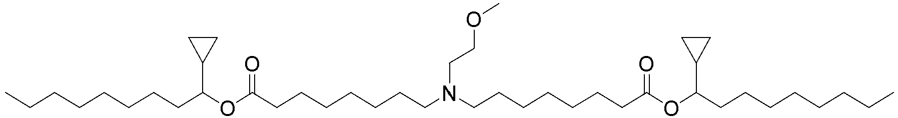 |
| L | 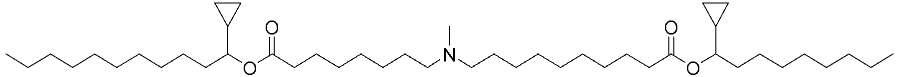 |
| M | 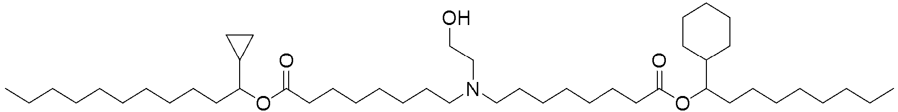 |
| N | 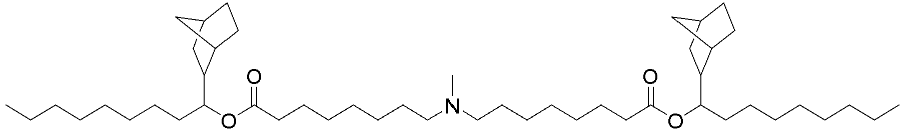 |
| O | 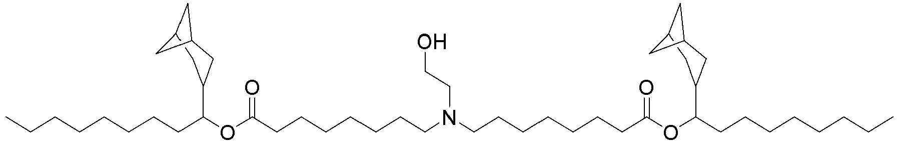 |

In an embodiment, the amount of the lipid in the composition for drug delivery of the present invention may be, based on the dry weight of the total composition, 5 wt% or more, 10 wt% or more, 15 wt% or more, 20 wt% or more, 25 wt% or more, 30 wt% or more, or 35 wt% or more, and it may also be 95 wt% or less, 90 wt% or less, 85 wt% or less, 80 wt% or less, 75 wt% or less, 70 wt% or less, or 65 wt% or less. If the amount of the lipid is too less, it may not be sufficient to form nanoparticles. To the contrary, if the amount of the lipid is too much, the size of the nanoparticle becomes too large, and thus the nanoparticle stability may be lowered and the rate of loss during filter sterilization may increase.

### Polymer

The composition for drug delivery of the present invention comprises lipid-polymer, amphiphilic block copolymer, or a combination thereof.

The lipid-polymer is a polymer having both hydrophilic and hydrophobic parts within the polymer molecule.

In an embodiment, the lipid-polymer may be a polymer in which one or more saturated or unsaturated hydrocarbon groups having 11 to 25 carbon atoms as a hydrophobic part are introduced into a hydrophilic block which is a hydrophilic part.

The hydrophilic block may be one or more selected from the group consisting of polyalkylene glycol, polyvinyl alcohol, polyvinyl pyrrolidone, polyacrylamide, and derivatives thereof.

More concretely, the hydrophilic block may be one or more selected from the group consisting of monomethoxypolyethylene glycol, monoacetoxypolyethylene glycol, polyethylene glycol, copolymer of polyethylene and propylene glycol, and polyvinylpyrrolidone.

In an embodiment, the number average molecular weight (g/mol) of the hydrophilic block may be 200 or more, 500 or more, 1,000 or more, or 2,000 or more, and it also may be 50,000 or less, 20,000 or less, 10,000 or less, or 5,000 or less, but it is not limited thereto.

Also, if necessary, the end of the hydrophilic block may be chemically combined with a functional group or ligand capable of reaching specific tissue or cell, or a functional group capable of promoting intracellular delivery, in order to control *in vivo* distribution of the nanoparticle carrier or to increase the efficiency of delivering the nanoparticle carrier into cell. The functional group or ligand may be one or more selected from the group consisting of monosaccharides, polysaccharides, vitamins, peptides, proteins, and antibodies to cell surface receptors. More concretely, the functional group or ligand may be one or more selected from the group consisting of anisamide, vitamin B9 (folic acid), vitamin B12, vitamin A, galactose, lactose, mannose, hyaluronic acid, RGD peptide, NGR peptide, transferrin, antibody to transferrin receptor, etc.

In an embodiment, the saturated or unsaturated hydrocarbon group having 11 to 25 carbon atoms, the hydrophobic part introduced into a hydrophilic block which is the hydrophilic part, may independently be selected from the group consisting of myristoyl, dimyristoyl, lauryl, myristyl, palmityl, stearyl, arachidyl, behenyl, lignoceryl, cerotyl, myristoleyl, palmitoleyl, sapienyl, oleyl, linoleyl, arachidonyl, eicosapentaenyl, erucyl, and docosahexaenyl.

Also, in an embodiment, in the lipid-polymer, the amount ratio of the hydrophilic part and the hydrophobic part may be such as 40 to 70 wt% and more specifically 50 to 60 wt% of the hydrophilic part, based on the weight of the polymer. If the ratio of the hydrophilic part is too small, the solubility of the polymer in water is low, making it difficult to form nanoparticles, and to the contrary, if it is too large, the hydrophilicity becomes too high, which may lower the stability of the nanoparticles.

In an embodiment of the present invention, the lipid-polymer may be a polyalkylene glycol (e.g., polyethylene glycol) into which saturated or unsaturated hydrocarbon group having 11 to 25 carbon atoms (e.g., myristyl group) has been introduced.

The amphiphilic block copolymer may be an A-B type block copolymer comprising a hydrophilic A block and a hydrophobic B block. In an aqueous environment, the A-B type block copolymer forms core-shell type polymer nanoparticle wherein the hydrophobic B block forms the core (inner wall) and the hydrophilic A block forms the shell (outer wall).

In an embodiment, the hydrophilic A block may be one or more selected from the group consisting of polyalkylene glycol, polyvinyl alcohol, polyvinyl pyrrolidone, polyacrylamide, and derivatives thereof.

More concretely, the hydrophilic A block may be one or more selected from the group consisting of monomethoxypolyethylene glycol (mPEG), monoacetoxypolyethylene glycol, polyethylene glycol, copolymer of polyethylene and propylene glycol, and polyvinylpyrrolidone.

In an embodiment, the number average molecular weight (g/mol) of the hydrophilic A block may be 200 or more, 500 or more, 1,000 or more, or 2,000 or more, and it also may be 50,000 or less, 20,000 or less, 10,000 or less, or 5,000 or less, but it is not limited thereto.

Also, if necessary, the end of the hydrophilic A block may be chemically combined with a functional group or ligand capable of reaching specific tissue or cell, or a functional group capable of promoting intracellular delivery, in order to control *in vivo* distribution of polymer nanoparticle carrier formed by the amphiphilic block copolymer and salt of polylactic acid or to increase the efficiency of delivering the nanoparticle carrier into cell. In an embodiment, the functional group or ligand may be one or more selected from the group consisting of monosaccharides, polysaccharides, vitamins, peptides, proteins, and antibodies to cell surface receptors. More concretely, the functional group or ligand may be one or more selected from the group consisting of anisamide, vitamin B9 (folic acid), vitamin B12, vitamin A, galactose, lactose, mannose, hyaluronic acid, RGD peptide, NGR peptide, transferrin, antibody to transferrin receptor, etc.

The hydrophobic B block is a biocompatible, biodegradable polymer, and in an embodiment, it may be one or more selected from the group consisting of polyester, polyanhydride, polyamino acid, polyorthoester and polyphosphazine.

More concretely, the hydrophobic B block may be one or more selected from the group consisting of polylactide (PLA), polyglycolide, polycaprolactone, polydioxan-2-one, copolymer of polylactide and polyglycolide, copolymer of polylactide and polydioxan-2-one, copolymer of polylactide and and polycaprolactone, and a copolymer of polyglycolide and polycaprolactone.

In an embodiment, the number average molecular weight (g/mol) of the hydrophobic B block may be 200 or more, 500 or more, 1,000 or more, or 1,700 or more, and it also may be 50,000 or less, 20,000 or less, 10,000 or less, or 6,000 or less, but it is not limited thereto.

For example, the number average molecular weight combination of the hydrophilic A block-hydrophobic B block may be 2,000-6,000, 2,000-5,400, 2,000-4,000, 2,000-3,000, 2,000-1,700, etc., but it is not limited thereto.

Also, in an embodiment, in order to increase the hydrophobicity of and thereby improve the stability of the nanoparticle, the hydrophobic B block may be modified by chemically combining the hydroxyl group at the end of the hydrophobic B block with tocopherol, cholesterol, or fatty acids having 10 to 24 carbons.

In an embodiment, in the amphiphilic block copolymer, the amount ratio of the hydrophilic block (A) and the hydrophobic block (B) may be such as 40 to 70 wt% and more specifically 50 to 60 wt% of the hydrophilic block (A), based on the weight of the copolymer. If the ratio of the hydrophilic block (A) is less than 40 wt%, the solubility of the polymer in water is low, making it difficult to form nanoparticles. Thus, in order for the copolymer to have sufficient water solubility to form nanoparticles, it is preferable that the ratio of the hydrophilic block (A) be 40% by weight or more. To the contrary, if the ratio of the hydrophilic block (A) is greater than 70 wt%, the hydrophilicity becomes too high, lowering the stability of the nanoparticles and making it difficult to use as a solubilizing composition of effective ingredient/lipid complex. Thus, considering the stability of the nanoparticles, it is preferable that the ratio of the hydrophilic block (A) be 70 wt% or less.

In an embodiment, the amount of the polymer, which is lipid-polymer, amphiphilic block copolymer, or a combination thereof, in the composition for drug delivery of the present invention may be, based on the dry weight of the total composition, 5 wt% or more, 7 wt% or more, 10 wt% or more, 12 wt% or more, 15 wt% or more, 17 wt% or more, or 20 wt% or more, and it may also be 90 wt% or less, 80 wt% or less, 70 wt% or less, 60 wt% or less, 50 wt% or less, or 40 wt% or less. If the amount of the polymer is too less, the size of the nanoparticle becomes too large, and thus the nanoparticle stability may be lowered and the rate of loss during filter sterilization may increase. To the contrary, if the amount of the polymer is too much, there is a concern that the amount of the effective ingredient that can be incorporated will become too less.

In the composition for drug delivery of the present invention, the effective ingredient is maintained in a state of being encapsulated within a nanoparticle structure formed by the polymer, which is lipid-polymer, amphiphilic block copolymer, or a combination thereof, and the lipid of the above formula 1, thereby improving stability in blood or body fluids.

In an embodiment, the particle size of the nanoparticle can be defined by Z-average value, and for example, it may be 800 nm or less, 600 nm or less, 500 nm or less, 400 nm or less, 300 nm or less, 200 nm or less, or 150 nm or less, and also may be 10 nm or more, 50 nm or more, or 100 nm or more. In an embodiment, the particle size of the nanoparticle defined by Z-average value may be, for example, 10 to 800 nm, 20 to 600 nm, 30 to 500 nm, 50 to 400 nm, or 80 to 300 nm.

In an embodiment, the relative amount of the polymer, which is lipid-polymer, amphiphilic block copolymer, or a combination thereof, to the lipid of the above formula 1 may be, based on 1 part by weight of the lipid of formula 1, 0.01 part by weight or more, 0.02 part by weight or more, 0.03 part by weight or more, 0.04 part by weight or more, or 0.05 part by weight or more, and it may also be 50 parts by weight or less, 49 parts by weight or less, 47 parts by weight or less, 45 parts by weight or less, 43 parts by weight or less, 41 parts by weight or less, 40 parts by weight or less, 39 parts by weight or less, or 37 parts by weight or less, but it is not limited thereto.

### Optional additive component

In an embodiment, in order to increase the efficiency of *in vivo* delivery of the effective ingredient, the composition for drug delivery of the present invention may further comprise fusogenic lipid.

In an embodiment, the fusogenic lipid may be one or a combination of two or more selected from the group consisting of phospholipid, cholesterol, and tocopherol.

Concretely, the phospholipid may be one or more selected from the group consisting of phosphatidylethanolamine (PE), phosphatidylcholine (PC) and phosphatidic acid. The phosphatidylethanolamine (PE), phosphatidylcholine (PC) and phosphatidic acid may be in a form combined with one or two C₁₀₋₂₄ fatty acids. The cholesterol and tocopherol include analogues, derivatives and metabolites of each of the cholesterol and tocopherol.

More concretely, the fusogenic lipid may be one or a combination of two or more selected from the group consisting of dilauroyl phosphatidylethanolamine, dimyristoyl phosphatidylethanolamine, dipalmitoyl phosphatidylethanolamine, distearoyl phosphatidylethanolamine, dioleoyl phosphatidylethanolamine, dilinoleoyl phosphatidylethanolamine, 1-palmitoyl-2-oleoyl phosphatidylethanolamine, 1,2-diphytanoyl-3-sn-phosphatidylethanolamine, Dilauroyl phosphatidylcholine, dimyristoyl phosphatidylcholine, dipalmitoyl phosphatidylcholine, distearoyl phosphatidylcholine, dioleoyl phosphatidylcholine, dilinoleoyl phosphatidylcholine, 1-palmitoyl-2-oleoyl phosphatidylcholine, 1,2-diphytanoyl-3-sn-phosphatidylcholine, dilauroyl phosphatidic acid, dimyristoyl Phosphatidic acid (dimyristoyl phosphatidic acid), dipalmitoyl phosphatidic acid, distearoyl phosphatidic acid, dioleoyl phosphatidic acid, dilinoleoyl phosphatidic acid, 1-palmitoyl-2-oleoyl phosphatidic acid, 1,2-diphytanoyl-3-sn-phosphatidic acid, cholesterol and tocopherol.

Still more concretely, the fusogenic lipid may be one or a combination of two or more selected from the group consisting of dioleoyl phosphatidylethanolamine (DOPE), 1,2-dipalmitoleoyl-sn-glycero-3-phosphocholine (DPPC), distearoyl phosphatidylcholine, 1,2-dioleoyl-sn-glycero-3-phosphocholine (DOPC), 1,2-dipalmitoleoyl-sn-glycero-3-phosphoethanolamine (DPPE), 1,2-dioleoyl-3-trimethylammonium-propane (DOTAP), cholesterol, and tocopherol.

In an embodiment of the present invention, the fusogenic lipid may be distearoyl phosphatidylcholine, cholesterol, or a combination thereof.

In an embodiment, the amount of the fusogenic lipid may be, based on the dry weight of the total composition, 1 wt% or more, 2 wt% or more, 3 wt% or more, 4 wt% or more, or 5 wt% or more, and it may also be 70 wt% or less, 65 wt% or less, 60 wt% or less, 55 wt% or less, 50 wt% or less, 45 wt% or less, 40 wt% or less, 35 wt% or less, 30 wt% or less, 25 wt% or less, or 20 wt% or less.

In an embodiment, the relative amount of the fusogenic lipid to the lipid of the above formula 1 may be, based on 1 part by weight of the lipid of formula 1, 0.05 part by weight or more, 0.06 part by weight or more, 0.07 part by weight or more, 0.08 part by weight or more, 0.09 part by weight or more, or 0.1 part by weight or more, and it may also be 6 parts by weight or less, 5.5 parts by weight or less, 5 parts by weight or less, 4.5 parts by weight or less, 4 parts by weight or less, or 3.8 parts by weight or less, but it is not limited thereto.

In an embodiment, in case of using phospholipid as the fusogenic lipid, its relative amount may be, based on 1 part by weight of the lipid of formula 1, 0.03 part by weight or more, 0.04 part by weight or more, 0.05 part by weight or more, or 0.06 part by weight or more, and it may also be 4 parts by weight or less, 3.9 parts by weight or less, 3.7 parts by weight or less, 3.5 parts by weight or less, 3.3 parts by weight or less, 3.1 parts by weight or less, 3 parts by weight or less, 2.9 parts by weight or less, or 2.7 parts by weight or less, but it is not limited thereto.

In an embodiment, in case of using cholesterol as the fusogenic lipid, its relative amount may be, based on 1 part by weight of the lipid of formula 1, 0.02 part by weight or more, 0.03 part by weight or more, or 0.04 part by weight or more, and it may also be 2.5 parts by weight or less, 2.3 parts by weight or less, 2.1 parts by weight or less, 2 parts by weight or less, 1.9 parts by weight or less, 1.7 parts by weight or less, 1.5 parts by weight or less, 1.3 parts by weight or less, or 1.1 parts by weight or less, but it is not limited thereto.

### Composition and preparation method thereof

The composition for drug delivery according to the present invention can be administered through routes of administration such as blood vessels, muscles, mucous membranes, subcutaneous, intradermal, oral, bone, transdermal, or local tissues, and can be formulated into various oral or parenteral formulations suitable for such routes of administration. Examples of the oral formulations include various ones such as tablets, capsules, powder formulations, liquid formulations, etc., and examples of the parenteral formulations include various ones such as eye drops, injections, etc., and in an embodiment, the composition may be an injection formulation. For example, when the composition according to the present invention is freeze-dried, it can be reconstituted with distilled water for injection, 0.9% physiological saline, 5% aqueous dextrose solution, etc. to produce an injection formulation.

The present invention also provides a method for preparing a composition for drug delivery, comprising the steps of: (a) preparing a solution in which a lipid represented by the above formula 1; and lipid-polymer, amphiphilic block copolymer, or a mixture thereof; are dissolved in a water-miscible organic solvent; and (b) to the solution prepared in step (a), adding effective ingredient selected from nucleic acid, polypeptide, virus, or combination thereof, and mixing them.

In an embodiment, step (a) can be performed in a solution under acidic condition.

In an embodiment, the water-miscible organic solvent in step (a) may be ethanol.

In an embodiment, step (b) may comprise: (b-1) a step of preparing a buffer solution containing the effective ingredient; and (b-2) a step of adding the buffer solution of the effective ingredient prepared in step (b-1) to the solution prepared in step (a), and mixing them.

In an embodiment, the mixing ratio of the buffer solution of the effective ingredient prepared in step (b-1) to the solution prepared in step (a) may be 1:1 to 1:5 in volume ratio, and more specifically, it may be 1:2 to 1:4.

In another embodiment, step (b) may comprise: (b-1) a step of adding the effective ingredient to the solution prepared in step (a); and (b-2) a step of adding a buffer solution to the resulting mixture of step (b-1) and mixing them.

In an embodiment, the method for preparing a composition for drug delivery may further comprise a step of adding a pH adjusting buffer, water for injection, or a combination thereof to the resulting mixture of step (b).

In another embodiment, the method for preparing a composition for drug delivery may further comprise a step of removing the solvent from the resulting mixture of step (b) and then adding a freeze-drying aid thereto and freeze-drying the resulting mixture.

The freeze-drying aid is added to help the freeze-dried composition maintain a cake shape or to help the composition dissolve uniformly within a short period of time during the reconstitution process after freeze-drying, and specifically, it may be one or more selected from the group consisting of lactose, mannitol, sorbitol, and sucrose. The amount of the freeze-drying aid may be 1 to 90 wt%, more specifically 10 to 60 wt%, based on the total dry weight of the freeze-dried composition.

The present invention will be explained below in more detail with reference to the following Examples. However, the Examples are only to illustrate the invention, and the scope of the present invention is not limited thereby in any manner.

### [EXAMPLES]

### Lipid Preparation Example 1

The compound of the following formula A was prepared as follows.

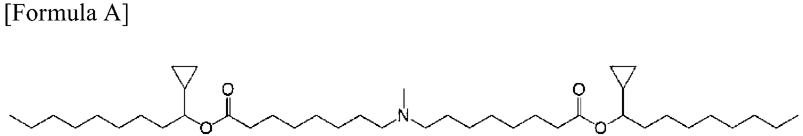

### (1) Synthesis of 1-cyclopropylnonan-1-ol

In a 2000 mL 3-neck round bottom flask (RBF), cyclopropanecarbaldehyde (35.0 g, 499 mmol, 1.00 eq) and tetrahydrofuran (THF) (700 mL) were added under a nitrogen environment and cooled to -65°C, and then octylmagnesium bromide (2 M, 375 mL, 1.50 eq) was added, and the mixture was stirred at -65°C for 2 hours. The reactor was heated to 15°C, then the reaction mixture was poured into a saturated NH₄Cl aqueous solution (500 mL), and the organic layer and aqueous layer were separated. The aqueous layer was extracted with ethyl acetate (EtOAc) (450 mL) (150 mL each, three times). The organic layers were collected, concentrated in vacuo, and purified using a silica column with petroleum ether:EtOAc = 50:1 → 0:1 to obtain 1-cyclopropylnonan-1-ol (87.5 g, 73.1%).

¹H NMR (400 MHz, CHLOROFORM-*d*): *δ* 2.93 - 2.81 (m, 1H), 1.61 (br d, 2H), 1.52 - 1.27 (m, 12H), 0.95 - 0.86 (m, 4H), 0.60 - 0.45 (m, 2H), 0.34 - 0.19 (m, 2H)

### (2) Synthesis of 1-cyclopropylnonyl 8-bromooctanoate

In a 1000 mL 3-neck RBF, 1-cyclopropylnonan-1-ol (30.0 g, 163 mmol, 1.00 eq), 8-bromooctanoic acid (72.6 g, 326 mmol, 2.00 eq), methylene chloride (DCM) (300 mL), 1-ethyl-3-(3-dimethylaminopropyl)carbodiimide (EDCI) (31.2 g, 163 mmol, 1.00 eq), and 4-dimethylaminopyridine (DMAP) (19.9 g, 163 mmol, 1.00 eq) were added, and the mixture was stirred at 25°C for 16 hours. The reaction mixture was concentrated in vacuo, and after adding silica powder thereto, it was purified using a silica column with petroleum ether:EtOAc = 10:1 → 50:1 to obtain 1-cyclopropylnonyl 8-bromooctanoate (22.8 g, 36.0%).

¹H NMR (400 MHz, CHLOROFORM-*d*): *δ* 4.29 (td, 1H), 3.59 - 3.31 (m, 2H), 2.32 (t, 2H), 1.94 - 1.75 (m, 2H), 1.73 - 1.60 (m, 4H), 1.49 - 1.25 (m, 18H), 1.03 - 0.93 (m, 1H), 0.90 (t, 3H), 0.61 - 0.24 (m, 4H)

### (3) Synthesis of the compound of formula A

In a 100 mL 3-neck flask, methylamine hydrochloride (173 mg, 2.57 mmol, 1.00 eq), ethanol (EtOH) (30 mL), N,N-diisopropylethylamine (DIEA) (1.66 g, 12.8 mmol, 5.00 eq), and 1-cyclopropylnonyl 8-bromooctanoate (3.00 g, 7.70 mmol, 3.00 eq) were added sequentially, and the mixture was stirred at 80°C for 72 hours. The reaction mixture was concentrated in vacuo, and after adding silica powder thereto, it was purified using a silica column with petroleum ether:EtOAc = 10:1 → 1:1 to obtain the compound of formula A (660 mg, 38.9%).

¹H NMR (400 MHz, CHLOROFORM-*d*): *δ* 4.29 (td, 2H), 2.32 (br t, 8H), 2.22 (s, 3H), 1.74 - 1.60 (m, 8H), 1.54 - 1.42 (m, 4H), 1.39 - 1.23 (m, 36H), 1.02 - 0.87 (m, 8H), 0.61 - 0.23 (m, 8H)

### Lipid Preparation Example 2

The compound of the following formula B was prepared as follows.

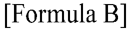

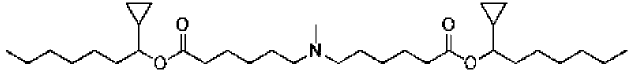

### (1) Synthesis of 1-cyclopropylheptan-1-ol

In a 2000 mL 3-neck RBF, cyclopropanecarbaldehyde (27.0 g, 385 mmol, 1.00 eq) and THF (500 mL) were added under a nitrogen environment, cooled to -65°C, and hexylmagnesium bromide (1 M, 500 mL, 1.30 eq) was slowly added. The mixture was stirred at -65°C for 3 hours, and then the temperature of the reactor was slowly increased to 25°C. Then, the mixture was poured into a saturated NH₄Cl aqueous solution (500 mL), and the organic layer and aqueous layer were separated. The aqueous layer was extracted with EtOAc (500 mL each, three times). The organic layers were collected and concentrated in vacuo, and the residue after concentration was purified using a silica column with petroleum ether:EtOAc = 50:1 → 10:1 to obtain 1-cyclopropylheptan-1-ol (46.0 g, 294 mmol, 76.4% yield) as a colorless oil.

¹H NMR (400 MHz, CHLOROFORM-*d*): δ 2.86 (td, 1H), 1.67 - 1.38 (m, 6H), 1.36 - 1.27 (m, 6H), 0.91 - 0.87 (m, 3H), 0.59 - 0.42 (m, 2H), 0.32 - 0.17 (m, 2H)

### (2) Synthesis of 1-cyclopropylheptyl 6-bromohexanoate

In a 500 mL 3-neck RBF, 1-cyclopropylheptan-1-ol (10.0 g, 63.9 mmol, 1.00 eq), 6-bromohexanoic acid (12.5 g, 63.9 mmol, 1.00 eq), DCM (100 mL), EDCI (15.9 g, 83.2 mmol, 1.30 eq), and DMAP (10.2 g, 83.2 mmol, 1.30 eq) were added, and the mixture was stirred at 25 °C for 16 hours. The reaction mixture was concentrated in vacuo, and purified using a silica column with petroleum ether:EtOAc = 50:1 → 10:1 to obtain 1-cyclopropylheptyl 6-bromohexanoate (7.50 g, 22.5 mmol, 35.1%)

¹H NMR: (400 MHz, CHLOROFORM-*d*): δ 4.28 (td, 1H), 3.55 (t, 1H), 3.42 (t, 1H), 2.39 - 2.26 (m, 2H), 1.97 - 1.76 (m, 2H), 1.71 - 1.60 (m, 4H), 1.57 - 1.42 (m, 3H), 1.33 - 1.28 (m, 6H), 0.91 - 0.86 (m, 5H), 0.63 - 0.51 (m, 1H), 0.50 - 0.41 (m, 1H), 0.40 - 0.32 (m, 1H), 0.30 - 0.22 (m, 1H)

### (3) Synthesis of the compound of formula B

In a 100 mL 3-neck RBF, 1-cyclopropylheptyl 6-bromohexanoate (500 mg, 1.00 eq) was placed, and methylamine in THF (2 M, 2 g, 42.8 eq) was added under nitrogen environment, and then the mixture was stirred at 50°C for 16 hours. To the stirred reaction mixture, a sodium carbonate (Na₂CO₃) aqueous solution (20 mL) was added and stirred for 2 hours to adjust the pH to 8. Then, extraction was performed with DCM (30 mL*3) and the organic layer was preserved. The preserved organic layer was concentrated in vacuo and purified using a silica column with DCM: methanol = 10:1 to obtain the compound of formula B (68 mg, 8.56%) as a yellow oil.

¹H NMR (400 MHz, CHLOROFORM-*d*) *δ* 4.27 (td, 2H), 2.43 - 2.16 (m, 10H), 1.75 - 1.62 (m, 8H), 1.55 - 1.43 (m, 5H), 1.40 - 1.20 (m, 22H), 1.00 - 0.91 (m, 2H), 0.90 - 0.85 (m, 4H), 0.62 - 0.50 (m, 2H), 0.49 - 0.33 (m, 4H), 0.30 - 0.21 (m, 2H)

### Lipid Preparation Example 3

The compound of the following formula C was prepared as follows.

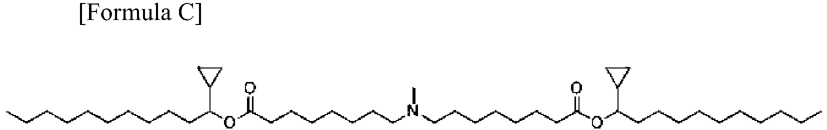

### (1) Synthesis of 1-cyclopropylundecan-1-ol

In a 2000 mL 3-neck RBF, cyclopropanecarbaldehyde (27.0 g, 385 mmol, 1.00 eq) and THF (270 mL) were added, and the mixture was purged with nitrogen three times, cooled to -60°C, and then decylmagnesium bromide (1 M, 501 mL, 1.30 eq) was added, and the mixture was stirred at -60 °C for 16 hours in a nitrogen environment. The reactor was heated to 25°C, and the mixture was poured into a saturated NH₄Cl aqueous solution (200 mL) to separate the organic and aqueous layers. The aqueous layer was extracted with EtOAc (100 mL each, 4 times), and the organic layer was collected, concentrated in vacuo and purified using a silica column with petroleum ether:EtOAc = 10:1 to obtain 1-cyclopropylundecan-1-ol (56.0 g, 68.5%).

¹H NMR (400 MHz, CHLOROFORM-*d*): *δ* 0.17 - 0.31 (m, 2 H) 0.43 - 0.57 (m, 2 H) 0.88 (t, 4 H) 1.26 (br s, 14 H) 1.37 - 1.49 (m, 2 H) 1.54 - 1.64 (m, 3 H) 2.85 (dt, 1 H)

### (2) Synthesis of 1-cyclopropylundecyl 8-bromooctanoate

In a 1000 mL 3-neck RBF, 1-cyclopropylundecan-1-ol (24.0 g, 113 mmol, 1.00 eq), 8-bromooctanoic acid (32.8 g, 147 mmol, 1.30 eq), DCM (300 mL), EDCI (26.0 g, 136 mmol, 1.20 eq), and DMAP (16.6 g, 136 mmol, 1.20 eq) were added, and the mixture was purged with nitrogen three times. The mixture was stirred at 25°C for 16 hours and then poured into water (200 mL), and the organic layer and the aqueous layer were separated. The aqueous layer was extracted with DCM (200 mL each, three times). The extracted organic layer was dried over Na₂SO₄, and the dried mixture was filtered and concentrated in vacuo. The residue after concentration was purified using a silica column with petroleum ether:EtOAc=10:1→1:1 to obtain 1-cyclopropylundecyl 8-bromooctanoate (11.0 g, 23.3%).

¹H NMR (400 MHz, CHLOROFORM-*d*): *δ* 0.22 - 0.30 (m, 1 H) 0.33 - 0.40 (m, 1 H) 0.42 - 0.49 (m, 1 H) 0.50 - 0.58 (m, 1 H) 0.85 - 1.00 (m, 4 H) 1.20 - 1.38 (m, 20 H) 1.41 - 1.49 (m, 2 H) 1.58 - 1.68 (m, 4 H) 1.73 - 1.91 (m, 2 H) 2.25 - 2.37 (m, 2 H) 3.35 - 3.56 (m, 2 H) 4.21 - 4.35 (m, 1 H)

### (3) Synthesis of the compound of formula C

In a 100 mL 3-neck RBF, 1-cyclopropylundecyl 8-bromooctanoate (3.00 g, 7.19 mmol, 1.00 eq) and methylamine solution (2 M in THF, 10.3 g, 331 mmol, 46.0 eq) were, and then the mixture was stirred at 50°C for 16 hours. After concentrating the mixture in the reactor in vacuo, the pH was adjusted by adding NaHCO₃ aqueous solution. Then, the mixture was extracted with DCM, and the extracted organic layer was concentrated in vacuo. The residue after concentration was purified using a silica column with DCM:methanol = 10:1 → 1:1 to obtain the compound of formula C (2.00 g, 63.0%) as a yellow oil.

¹H NMR (400 MHz, CHLOROFORM-*d*) *δ* 0.21 - 0.30 (m, 2 H) 0.37 (dq, 2 H) 0.42 - 0.49 (m, 2 H) 0.50 - 0.58 (m, 2 H) 0.84 - 0.98 (m, 8 H) 1.24 - 1.36 (m, 44 H) 1.51 (br s, 4 H) 1.59 - 1.68 (m, 8 H) 2.23 - 2.33 (m, 7 H) 2.34 - 2.44 (m, 4 H) 4.21 - 4.33 (m, 2 H)

### Lipid Preparation Example 4

The compound of the following formula D was prepared as follows.

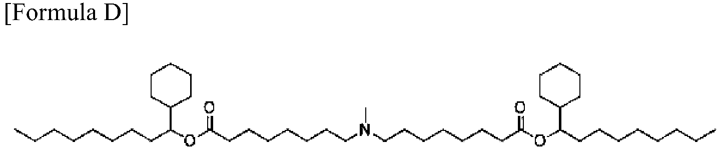

### (1) Synthesis of 1-cyclohexylnonan-1-ol

In a 1000 mL 3-neck RBF, cyclohexanecarbaldehyde (43.0 g, 383 mmol, 1.00 eq) and THF (430 mL) were added, and the mixture was purged three times with nitrogen, cooled to -65 °C, and then octylmagnesium bromide (1 M in THF, 498 mL, 1.30 eq) was added, and the mixture was stirred at -65 °C for 1 hour in a nitrogen environment. The reactor was heated to 25°C, and the mixture was poured into a saturated NH₄Cl aqueous solution (700 mL) to separate the organic layer and the aqueous layer. The aqueous layer was extracted with EtOAc (400 mL each, three times), the organic layers were collected, concentrated in vacuo, and purified using a silica column with petroleum ether:EtOAc = 10:1→1:10 to obtain 1-cyclohexylnonan-1-ol (12.0 g, 53.0 mmol, 13.8%) as a colorless oil.

¹HNMR (400 MHz, CHLOROFORM-*d*): *δ* 3.41 - 3.31 (m, 1H), 1.84 - 1.73 (m, 3H), 1.70 - 1.63 (m, 2H), 1.48 (br d, 3H), 1.36 - 1.21 (m, 15H), 1.18 - 1.00 (m, 3H), 0.94 - 0.84 (m, 3H)

### (2) Synthesis of 1-cyclohexylnonyl 8-bromooctanoate

In a 250 mL 3-neck RBF, 1-cyclohexylnonan-1-ol (7.00 g, 30.9 mmol, 1.00 eq) was placed, and DCM (70 mL) was added. Then, 8-bromooctanoic acid (8.28 g, 37.1 mmol, 1.20 eq), EDCI (7.11 g, 37.1 mmol, 1.20 eq), DMAP (755 mg, 6.18 mmol, 0.20 eq), and Et3N (6.26 g, 61.8 mmol, 2.00 eq) were added and mixed. The mixture was stirred at 25°C for 16 hours, and purged with nitrogen three times. The reaction mixture was filtered using a celite plug, and the filtrate was concentrated in vacuo. The residue after concentration was purified using a silica column with petroleum ether:EtOAc = 10:1 → 1:100 to give 1-cyclohexylnonyl 8-bromooctanoate (3.50 g, 8.11 mmol, 26.2%) as a yellow oil.

¹HNMR (400 MHz, CHLOROFORM-*d*): *δ* 4.07 (t, 2H), 3.41 (t, 2H), 2.30 (t, 2H), 1.91 - 1.80 (m, 2H), 1.67 - 1.58 (m, 4H), 1.47 - 1.41 (m, 2H), 1.37 - 1.26 (m, 16H), 0.91 - 0.87 (m, 3H)

### (3) Synthesis of the compound of formula D

In a 100 mL 3-neck RBF, 1-cyclohexylnonyl 8-bromooctanoate (1.50 g, 3.48 mmol, 1.00 eq) was placed, and methylamine in THF (CH₃NH₂ in THF) (15.6 g, 151 mmol, 30% purity, 43.5 eq) was added. The mixture was purged with nitrogen three times and stirred at 50°C for 16 hours. After stirring, the reaction mixture was filtered, and the filtrate was concentrated in vacuo. The residue after concentration was purified using a silica column with DCM:MeOH = 100:1 → 10:1 to give the compound of formula D (0.13 g, 178 µmol, 5.11% yield) as a yellow oil.

¹H NMR (400 MHz, CHLOROFORM-*d*) *δ* 4.81 - 4.69 (m, 2H), 2.30 (br t, 8H), 2.22 (br s, 3H), 1.76 - 1.71 (m, 4H), 1.69 - 1.60 (m, 12H), 1.53 - 1.43 (m, 10H), 1.35 - 1.22 (m, 40H), 1.05 - 0.97 (m, 4H), 0.88 (br t, 6H)

### Lipid Preparation Example 5

The compound of the following formula E was prepared as follows.

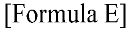

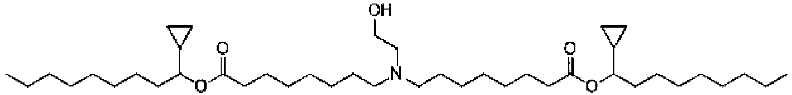

### (1) Synthesis of 1-cyclopropylnonan-1-ol

In a 1000 mL 3-neck RBF, cyclopropanecarbaldehyde (46.0 g, 656 mmol, 1.00 eq) was placed, THF was added, and then octylmagnesium bromide (2.00 M, 492 mL, 1.50 eq) was added under a nitrogen environment. The mixture was stirred at -65°C for 3 hours, and then saturated NH₄Cl aqueous solution (700 mL) was poured thereto at 15°C, and the organic layer and the aqueous layer were separated. The aqueous layer was additionally extracted with EtOAc (200 mL x 3). The organic layers were collected, concentrated in vacuo, and purified using a silica column with petroleum ether:EtOAc = 100:1 to obtain 1-cyclopropylnonan-1-ol (59.5 g, 49.2%) as a colorless oil.

¹H NMR (400 MHz, CHLOROFORM-*d*): *δ* 2.63 (td, 1H), 1.47 - 1.31 (m, 5H), 1.29 - 1.09 (m, 6H), 0.73 - 0.61 (m, 6H), 0.37 - 0.19 (m, 3H), 0.10 - 0.06 (m, 3H)

### (2) Synthesis of 1-cyclopropylnonyl 8-bromooctanoate

In a 1000 mL 3-neck RBF, DCM (600 mL), 1-cyclopropylnonan-1-ol (59.5 g, 325 mmol, 1.00 eq), 8-bromooctanoic acid (94.4 g, 423 mmol, 1.30 eq), EDCI (93.6 g, 488 mmol, 1.50 eq), DMAP (39.8 g, 326 mmol, 1.00 eq), and TEA (32.9 g, 326 mmol, 45.3 mL, 1.00 eq) were added, and the mixture was stirred at 25°C for 16 hours under a nitrogen environment. The reaction mixture was concentrated in vacuo to obtain a residue. The obtained residue was purified using a silica column with petroleum ether:EtOAc = 100:1 → 1:1. Through this process, 1-cyclopropylnonyl 8-bromooctanoate (21.0 g, 53.9 mmol, 16.6% yield) was obtained as pale yellow oil.

¹H NMR (400 MHz, CHLOROFORM-*d*): *δ* 0.05 - 0.16 (m, 2 H) 0.16 - 0.34 (m, 2 H) 0.57 - 0.67 (m, 2 H) 0.67 -0.80 (m, 1 H) 1.02 (br s, 9 H) 1.05 (br s, 2 H) 1.07 - 1.15 (m, 6 H) 1.16 - 1.29 (m, 2 H) 1.34 - 1.44 (m, 4 H) 1.48 - 1.66(m, 2 H) 2.05 (t, 2 H) 3.06 - 3.33 (m, 2 H) 3.97 - 4.07 (m, 1 H)

### (3) Synthesis of the compound of formula E

In a 100 mL 3-neck RBF, 1-cyclopropylnonyl 8-bromooctanoate (4.78 g, 12.3 mmol, 2.50 eq), 2-aminoethanol (MEA) (0.30 g, 4.91 mmol, 1.00 eq), and Na₂CO₃ (521 mg, 4.91 mmol, 1.00 eq) were added in EtOH (5 mL), and the mixture was purged with nitrogen three times. The mixture was stirred at 95°C for 16 hours. The mixture in the reactor was concentrated in vacuo to obtain a residue. The residue was then purified using a silica column with DCM:MeOH = 100:1 → 10:1 to obtain the compound of formula E (1.00 g, 1.47 mmol, 30.0% yield) as a yellow oil.

¹H NMR (400 MHz, CHLOROFORM-*d*): *δ* = 4.27 (td, 2H), 3.53 (t, 2H), 2.57 (t, 2H), 2.47 - 2.40 (m, 4H), 2.30 (t, 4H), 1.70 - 1.57 (m, 10H), 1.48 - 1.39 (m, 4H), 1.36 - 1.26 (m, 33H), 0.95 (dt, 2H), 0.89 (t, 6H), 0.60 - 0.42 (m, 4H), 0.41 - 0.22 (m, 4H)

### Lipid Preparation Example 6

The compound of the following formula F was prepared as follows.

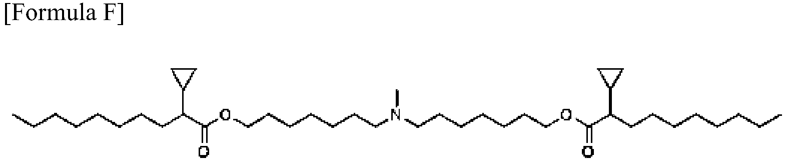

### (1) Synthesis of 2-cyclopropyldecanoic acid

In a 2000 mL 3-neck RBF, 2-cyclopropylacetic acid (25.0 g, 250 mmol, 1.00 eq) was added, followed by addition of THF (250 mL) and cooling with nitrogen. Then, sodium hydride (NaH) (11.0 g, 275 mmol, 60% purity, 1.10 eq) was added and the mixture was stirred at 0°C for 30 min. Then, lithium diisopropylamide (LDA) (2 M, 137 mL, 1.10 eq) was added at the same temperature and conditions, and the mixture was stirred for 30 min. Then, 1-iodooctane (60.0 g, 250 mmol, 1.00 eq) was added at 25°C, and the mixture was stirred at 45°C for 12 hours in a nitrogen environment. The reaction mixture was neutralized with 100 mL of water and 1 M HCl (600 mL, pH = 4), and then extracted with EtOAc (300 mL * 3). The extracted organic layer was dried over anhydrous Na₂SO₄ and concentrated in vacuo. The residue after concentration was purified using a silica column with petroleum ether:EtOAc = 20:1 → 5:1 to obtain 2-cyclopropyldecanoic acid (42.6 g, 201 mmol, 80.4% yield) as a yellow oil.

¹H NMR (400 MHz, CHLOROFORM-d): *δ* 11.88 (s, 1H), 1.84 - 1.70 (m, 1H), 1.70 - 1.54 (m, 2H), 1.42 - 1.19 (m, 12H), 1.00 - 0.81 (m, 4H), 0.63 - 0.45 (m, 2H), 0.32 (qd, 1H), 0.23 - 0.10 (m, 1H)

### (2) Synthesis of 7-bromoheptyl 2-cyclopropyldecanoate

In a 2000 mL 3-neck RBF, 2-cyclopropyldecanoic acid (10.0 g, 47.1 mmol, 1.00 eq), 7-bromoheptan-1-ol (11.0 g, 56.5 mmol, 1.20 eq), EDCI (11.7 g, 61.2 mmol, 1.30 eq), and DMAP (5.75 g, 47.1 mmol, 1.00 eq) were added together with DCM (100 mL), and the mixture was purged with nitrogen three times. The mixture was stirred at 25°C for 16 hours in a nitrogen environment, the reaction mixture was warmed to 25°C, and then poured into water (100 mL) to separate the organic layer and the aqueous layer. The aqueous layer was extracted with DCM (100 mL each, 3 times). The organic layers were collected, concentrated in vacuo, dried over anhydrous Na₂SO₄, and filtered. The filtered residue was purified using a silica column with petroleum ether:EtOAc = 20:1 → 5:1 to obtain 7-bromoheptyl 2-cyclopropyldecanoate (9.40 g, 24.1 mmol, 51.2%) as pale yellow oil.

¹H NMR (400 MHz, CHLOROFORM-*d*): *δ* 4.10 (t, *J* = 6.6 Hz, 2H), 3.42 (t, 2H), 1.89 - 1.84 (m, 1H), 1.81 - 1.51 (m, 6H), 1.50 - 1.35 (m, 6H), 1.27 (br s, 12H), 0.89 (t, 4H), 0.61 - 0.37 (m, 2H), 0.24 (s, 1H), 0.13 (qd, 1H)

### (3) Synthesis of 7-(methylamino)heptyl 2-cyclopropyldecanoate

In a 100 mL 3-neck RBF, 7-bromoheptyl 2-cyclopropyldecanoate (4.00 g, 10.3 mmol, 1.00 eq) and methylamine (2 M in THF, 185 mL, 36.0 eq) were added, and the mixture was stirred at 50°C for 16 hours in a nitrogen atmosphere. The stirred reaction mixture was concentrated in vacuo, extracted with DCM (30 mL*3) and the organic layer was preserved. The preserved organic layer was concentrated in vacuo and purified using a silica column with DCM:methanol = 20:1 → 10:1 to obtain 7-(methylamino)heptyl 2-cyclopropyldecanoate (0.45 g, 1.33 mmol, 12.9%) as a yellow oil.

¹H NMR (400 MHz, CHLOROFORM-*d*): *δ* 4.19 - 3.97 (m, 2H), 2.59 (t, 2H), 2.45 (s, 3H), 1.79 - 1.69 (m, 1H), 1.67 - 1.46 (m, 6H), 1.43 - 1.19 (m, 18H), 0.88 (t, 4H), 0.60 - 0.40 (m, 2H), 0.30 - 0.06 (m, 2H)

### (4) Synthesis of the compound of formula F

In a 50 mL 3-neck RBF, 7-(methylamino)heptyl 2-cyclopropyldecanoate (0.40 g, 1.18 mmol, 1.00 eq) was placed, and 7-bromoheptyl 2-cyclopropyldecanoate (0.60 g, 1.53 mmol, 1.30 eq) and Na₂CO₃ (0.25 g, 2.36 mmol, 2.00 eq) in dioxane (2 mL) were added. The mixture was stirred at 100°C for 16 hours, and then cooled and poured into water (5 mL) to separate the organic layer and the aqueous layer. The aqueous layer was extracted with EtOAc (5 mL x 3), and the organic layers were collected and concentrated in vacuo, and then washed with saturated Na₂CO₃ aqueous solution (5 mL), dried over anhydrous Na₂SO₄, and filtered. After concentration, the residue was purified using a silica column with DCM:methanol = 20:1 → 10:1 to obtain the compound of formula F (0.36 g, 556 µmol, 47.2%) as pale yellow oil.

¹H NMR: (400 MHz, CHLOROFORM-*d*) *δ* 4.09 (dt, 4H), 2.72 - 2.07 (m, 7H), 1.73 (br dd, 2H), 1.67 - 1.50 (m, 12H), 1.40 - 1.23 (m, 36H), 0.88 (br t, 8H), 0.59 - 0.50 (m, 2H), 0.45 (s, 2H), 0.24 (s, 2H), 0.13 (br d, 2H)

### Lipid Preparation Example 7

The compound of the following formula G was prepared as follows.

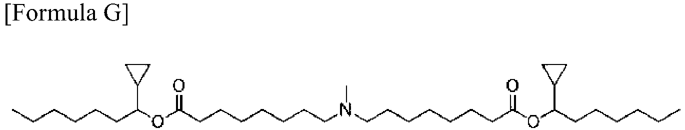

### (1) Synthesis of 1-cyclopropylheptan-1-ol

In a 2000 mL 3-neck RBF, cyclopropanecarbaldehyde (27.0 g, 385 mmol, 1.00 eq) was placed together with THF (500 mL). To this mixture, hexylmagnesium bromide (1 M in THF, 500 mL, 1.30 eq) was slowly added at 0°C. The mixture was stirred at 25°C for 4 hours. The reaction mixture was poured into a saturated NH₄Cl aqueous solution, and the organic layer and aqueous layer were separated. The aqueous layer was extracted with EtOAc (500 mL*3), and the organic layers were collected and concentrated in vacuo. The residue after concentration was purified using a silica column with petroleum ether:EtOAc = 50:1 → 10:1 to give 1-cyclopropylheptan-1-ol (46 g, 294.37 mmol, 76.4% yield) as a colorless oil.

¹H NMR (400 MHz, CHLOROFORM-*d*): *δ* 2.86 (td, 1H), 1.67 - 1.38 (m, 6H), 1.36 - 1.27 (m, 6H), 0.91 - 0.87 (m, 3H), 0.59 - 0.42 (m, 2H), 0.32 - 0.17 (m, 2H)

### (2) Synthesis of 1-cyclopropylheptyl 8-bromooctanoate

In a 2000 mL 3-neck RBF, 1-cyclopropylheptan-1-ol (10.0 g, 63.9 mmol, 1.00 eq) and 8-bromooctanoic acid (12.5 g, 63.9 mmol, 1.00 eq) in DCM (100 mL) were added. Then, DMAP (10.2 g, 83.19 mmol, 1.30 eq) and EDCI (15.9 g, 83.2 mmol, 1.30 eq) were added to the mixture. The mixture was stirred at 25°C for 16 hours. The reaction mixture was poured into H₂O (100 mL) and extracted, and then extracted again with DCM (100 mL*3). The organic layer was preserved and concentrated in vacuo. The residue after concentration was purified using a silica column with petroleum ether:EtOAc = 50:1 → 10:1 to obtain 1-cyclopropylheptyl 8-bromooctanoate (7.50 g, 22.5 mmol, 35.1% yield) as a colorless oil.

¹H NMR (400 MHz, CHLOROFORM-*d*): *δ* 4.28 (td, 1H), 3.59 - 3.36 (m, 2H), 2.35 - 2.26 (m, 2H), 1.92 - 1.72 (m, 2H), 1.69 - 1.60 (m, 4H), 1.50 - 1.41 (m, 2H), 1.37 - 1.24 (m, 12H), 1.01 - 0.84 (m, 4H), 0.62 - 0.50 (m, 1H), 0.50 - 0.41 (m, 1H), 0.37 (td, 1H), 0.26 (qd, 1H)

### (3) Synthesis of the compound of formula G

In a 500 mL 3-neck RBF, MeNH₂ (2 M in THF, 200.76 mL, 48.4 eq) was placed, and 1-cyclopropylheptyl 8-bromooctanoate (3.00 g, 8.30 mmol, 1.00 eq) was added to the flask under a nitrogen environment. The mixture was stirred at 80°C for 16 hours, and then the solvent was evaporated. The residue after evaporation was purified using a silica column with petroleum ether:EtOAc = 50:1 → 10:1 to obtain the compound of formula G (0.50 g, 844 µmol, 10.2% yield) as a yellow oil.

¹H NMR (400 MHz, CHLOROFORM-*d*): *δ* 0.26 (dq, 2 H) 0.34 - 0.40 (m, 2 H) 0.42 - 0.49 (m, 2 H) 0.50 - 0.58 (m, 2 H) 0.89 (br t, 6 H) 0.93 - 0.98 (m, 2 H) 1.26 - 1.34 (m, 26 H) 1.47 (br s, 4 H) 1.57 - 1.70 (m, 10 H) 2.13 - 2.41 (m, 11 H) 4.27 (dt, 2 H)

### Lipid Preparation Example 8

The compound of the following formula H was prepared. The synthesis method was the same as in Lipid Preparation Example 5 above, but 'cyclopropanecarbaldehyde' was replaced with 'cyclopentanecarbaldehyde,' and the same molar equivalents were used.

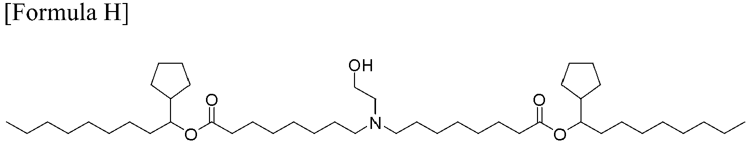

¹H NMR (400 MHz, CHLOROFORM-*d*): *δ* = 4.26 (td, 2H), 3.54 (t, 2H), 2.55 (t, 2H), 2.50 - 2.41 (m, 4H), 2.28 (t, 4H), 1.70 - 1.56 (m, 10H), 1.49 - 1.39 (m, 4H), 1.35 - 1.21 (m, 49H), 0.95 (dt, 2H), 0.89 (t, 6H)

### Lipid Preparation Example 9

The compound of the following formula I was prepared. The synthesis method was the same as in Lipid Preparation Example 5 above, but 'cyclopropanecarbaldehyde' was replaced with 'cyclopentanecarbaldehyde' and 'octylmagnesium bromide' was replaced with '7-methyloctylmagnesium bromide,' and the same molar equivalents were used.

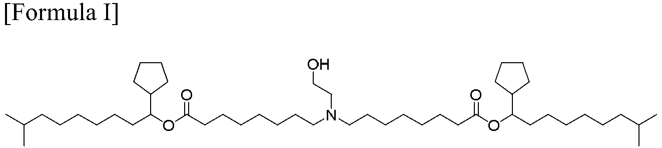

¹H NMR (400 MHz, CHLOROFORM-*d*): *δ* = 4.28 (td, 2H), 3.50 (t, 2H), 2.58 (t, 2H), 2.49 - 2.40 (m, 4H), 2.30 (t, 4H), 1.71 - 1.60 (m, 10H), 1.50 - 1.41 (m, 4H), 1.35 - 1.20 (m, 49H), 0.97 (dt, 2H), 0.92 (t, 6H)

### Lipid Preparation Example 10

The compound of the following formula J was prepared. The synthesis method was the same as in Lipid Preparation Example 7 above, but 'hexylmagnesium bromide' was replaced with '(3,7-dimethyloxyl)magnesium bromide,' and the same molar equivalents were used.

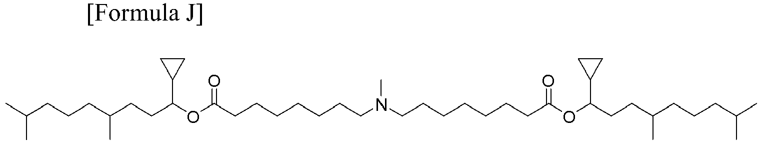

¹H NMR (400 MHz, CHLOROFORM-*d*): 4.19 (dt, 2 H), 2.41 - 2.15 (m, 11 H), 1.71 - 1.51 (m, 10 H), 1.47 (br s, 4 H), 1.33 - 1.25 (m, 22 H), 0.98 - 0.93 (m, 2 H), 0.91 - 0.88 (m, 18H), 0.58 - 0.50 (m, 2 H), 0.49 - 0.42 (m, 2 H), 0.40 - 0.34 (m, 2 H), *δ* 0.26 (dq, 2 H)

### Lipid Preparation Example 11

The compound of the following formula K was prepared. The synthesis method was the same as in Lipid Preparation Example 5 above, but '2-aminoethanol' was replaced with '2-methoxyethan-1-amine,' and the same molar equivalents were used.

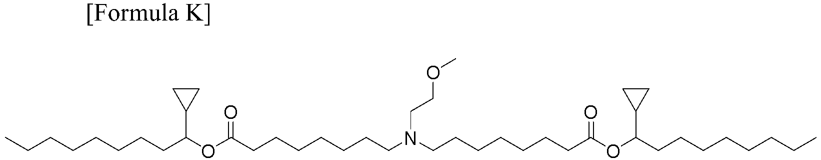

¹H NMR (400 MHz, CHLOROFORM-*d*): *δ* = 4.25 (td, 2H), 3.69 (t, 2H), 3.35 (s, 3H), 2.60 (t, 2H), 2.45 - 2.39 (m, 4H), 2.38 (t, 4H), 1.72 - 1.57 (m, 10H), 1.43 - 1.34 (m, 4H), 1.30 - 1.25 (m, 33H), 0.95 (dt, 2H), 0.88 (t, 6H), 0.60 - 0.42 (m, 4H), 0.41 - 0.22 (m, 4H)

### Lipid Preparation Example 12

The compound of the following formula L was prepared. The synthetic method was similar to Lipid Preparation Example 3 above. '1-cyclopropylundecyl 8-bromooctanoate' and '1-cyclopropylnonyl 10-bromodecanoate' were each prepared, and then reacted with 'methylamine' to prepare a compound of formula L.

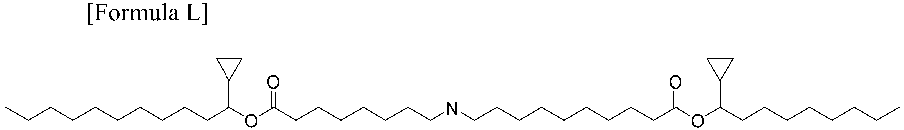

¹H NMR (400 MHz, CHLOROFORM-*d*): *δ* 4.28 (td, 2H), 2.41 (br t, 8H), 2.22 (s, 3H), 1.80 - 1.66 (m, 8H), 1.50 - 1.39 (m, 4H), 1.39 - 1.23 (m, 48H), 1.02 (m, 2H), 0.87 (m, 6H), 0.61 - 0.22 (m, 8H)

### Lipid Preparation Example 13

The compound of the following formula M was prepared. The synthetic method was similar to Lipid Preparation Example 5 above. '1-cyclopropylundecyl 8-bromooctanoate' and '1-cyclohexylnonyl 8-bromooctanoate' were each prepared, and then reacted with '2-aminoethanol' to prepare a compound of formula M.

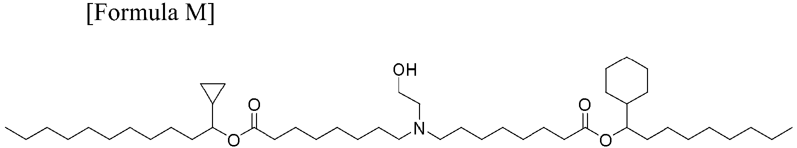

¹H NMR (400 MHz, CHLOROFORM-*d*): *δ* = 4.30-4.27 (m, 2H), 3.52 (t, 2H), 2.57 (t, 2H), 2.47 - 2.41 (m, 4H), 2.31-2.29 (m, 4H), 1.70 - 1.57 (m, 8H), 1.48 - 1.41 (m, 4H), 1.36 - 1.26 (m, 57H), 0.94 (dt, 1H), 0.88 (t, 6H), 0.60 - 0.42 (m, 2H), 0.41 - 0.22 (m, 2H)

### Lipid Preparation Example 14

The compound of the following formula N was prepared. The synthesis method was the same as in Lipid Preparation Example 1 above, but 'cyclopropanecarbaldehyde' was replaced with 'bicyclo[2.2.1]heptane-2-carbaldehyde,' and the same molar equivalents were used.

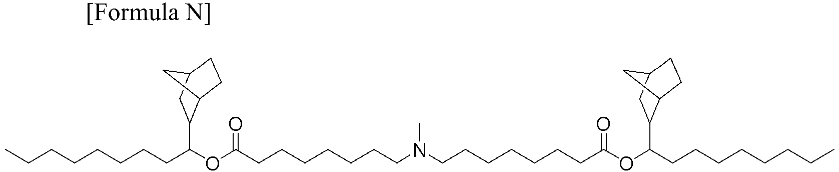

¹H NMR (400 MHz, CHLOROFORM-*d*): *δ* 4.35 (td, 2H), 2.35-2.29 (m, 8H), 2.20 (s, 3H), 2.18 - 2.15 (m, 2H), 1.75 - 1.66 (m, 8H), 1.54 - 1.23 (m, 60H), 0.89 (t, 6H)

### Lipid Preparation Example 15

The compound of the following formula O was prepared. The synthesis method was the same as in Lipid Preparation Example 5 above, but 'cyclopropanecarbaldehyde' was replaced with 'bicyclo[3.1.1]heptane-3-carbaldehyde,' and the same molar equivalents were used.

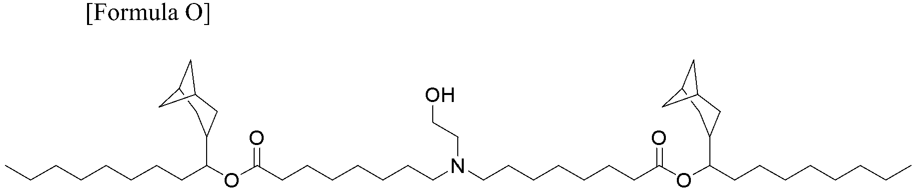

¹H NMR (400 MHz, CHLOROFORM-*d*): *δ* = 4.40 (td, 2H), 3.54 (t, 2H), 2.56 (t, 2H), 2.47 - 2.40 (m, 4H), 2.30 (t, 4H), 2.22 (m, 2H), 1.71 - 1.58 (m, 10H), 1.48 - 1.26 (m, 54H), 0.99 - 0.79 (m, 10H)

### [Preparation of composition and test of effective ingredient delivery to tissue]

### Example 1: Preparation of composition for drug delivery using the lipid of Preparation Example 1 and lipid-polymer, and test of drug delivery

### (1) Preparation of solutions for each component

The components shown in Table 1 below were dissolved in each dilution solvent to prepare their solutions at the concentrations shown in Table 1 below. When dissolving, a bath sonicator was used for about 5-10 minutes, and the solutions were used after visually confirming that there were no undissolved particles. For dioleoyl phosphatidylethanolamine (DOPE) and cholesterol, the solutions were incubated in an oven at 65°C for about 5 minutes and used in the test after visually confirming that there was no precipitation.

**[Table 1]**

| No. | Components | Dilution solvents | Concentration for use |
|---|---|---|---|
| 1 | mRNA | RNAse Free water | 1 mg/mL |
| 2 | Lipid of Preparation Example 1 | Ethanol 100% | 10-20 mg/mL |
| 3 | DOPE(1,2-dioleoyl-sn-glycero-3-phosphoethanolamine) | Ethanol 100% | 10-20 mg/mL |
| 4 | Cholesterol | Ethanol 100% | 10-20 mg/mL |
| 5 | DMG-PEG(1,2-dimyristoyl-rac-glycero-3-methoxypolyethylene glycol-2000) | Ethanol 100% | 5-20 mg/mL |

### (2) Mixing of raw materials

The required amounts of the components were taken and mixed in order to meet the N/P ratio (amine group of lipid component/phosphate group of mRNA) of 6 and the ratio of Lipid of Preparation Example 1:DOPE:cholesterol:DMG-PEG as shown in Table 2 below. Ethanol was added to the ethanol layer so that the molecular total of all components was 6.25-12.5 mM, and the aqueous phase and ethanol phase were mixed maintaining a ratio of 3:1. After mixing, in order to lower the total ethanol content, a buffer exchange was performed as follows: By using Amicon-Ultra tube filter (Merk Millipore, UFC505096 or UFC805024, pore size: 50K or 100K, volume: 0.5 mL or 4 mL or 15 mL), the buffer was exchanged through centrifuge at 4,000 rpm, concentration, and dilution with PBS.

The concrete procedure is as follows:
1) Two autoclaved tubes were prepared (tubes (A) and (B)).
2) In tube (A), Lipid of Preparation Example 1, DOPE, cholesterol, and DMG-PEG in molar quantities calculated according to the experimental conditions were sequentially added and mixed by vortexing.
3) In the ethanol phase, when necessary, ethanol was added so that the molecular total of all components was within 6.25-12.5 mM.
4) In tube (B), mRNA and 20 mM sodium acetate buffer (pH 4.6) were mixed. At that time, the ratio was calculated and added so that the aqueous phase was total three times the amount of the ethanol phase.
5) Mixing of Tube (A) and Tube (B) was performed using a Microfluidics (Ignite, Precision Nanosystem) device. Microfluidics operating conditions were FRR (Flow Rate Ratio) of C:R=3:1 and TRR (Total Flow Rate) of 12 mL/min.
6) The resulting mixture from step 5) was centrifuged at 4,000 rpm using an Amicon-Ultra tube filter (50K), and the process of concentration and dilution was repeated to remove excess ethanol, and then concentrated to a final x mg/ml (theoretical concentration).
7) Once the formulation was concentrated to the desired concentration, it was sterilized using a 0.22 µm pore size filter.

**[Table 2]**

| | Effective ingredient | Lipid | Fusogenic lipid | | Polymer |
|---|---|---|---|---|---|
| Comp. Example | mRNA | SM102 | DSPC | Cholesterol | DMG-PEG |
| 1 | 1 mg | 11.4 mg | 2.5 mg | 4.8 mg | 1.2 mg |
| Example | mRNA | Lipid of Prep. Ex. 1 | DOPE | Cholesterol | DMG-PEG |
| 1-1 | 1 mg | 12.0 mg | 4.6 mg | 14.0 mg | 2.3 mg |
| 1-2 | 1 mg | 12.0 mg | 9.2 mg | 11.6 mg | 2.3 mg |
| 1-3 | 1 mg | 12.0 mg | 13.8 mg | 9.2 mg | 2.3 mg |
| 1-4 | 1 mg | 12.0 mg | 18.4 mg | 6.8 mg | 2.3 mg |
| 1-5 | 1 mg | 12.0 mg | 23.0 mg | 4.4 mg | 2.3 mg |
| 1-6 | 1 mg | 12.0 mg | 27.7 mg | 2.0 mg | 2.3 mg |
| 1-7 | 1 mg | 12.0 mg | 3.5 mg | 8.7 mg | 1.7 mg |
| 1-8 | 1 mg | 12.0 mg | 6.9 mg | 6.9 mg | 1.7 mg |
| 1-9 | 1 mg | 12.0 mg | 10.4 mg | 5.1 mg | 1.7 mg |
| 1-10 | 1 mg | 12.0 mg | 13.8 mg | 3.3 mg | 1.7 mg |
| 1-11 | 1 mg | 12.0 mg | 17.3 mg | 1.5 mg | 1.7 mg |
| 1-12 | 1 mg | 12.0 mg | 5.5 mg | 4.1 mg | 1.4 mg |
| 1-13 | 1 mg | 12.0 mg | 8.3 mg | 2.7 mg | 1.4 mg |
| 1-14 | 1 mg | 12.0 mg | 11.1 mg | 1.2 mg | 1.4 mg |
| 1-15 | 1 mg | 12.0 mg | 2.3 mg | 3.4 mg | 1.2 mg |
| 1-16 | 1 mg | 12.0 mg | 4.6 mg | 2.2 mg | 1.2 mg |
| 1-17 | 1 mg | 12.0 mg | 2.0 mg | 1.9 mg | 1.0 mg |
| 1-18 | 1 mg | 12.0 mg | 0 mg | 28.0 mg | 3.0 mg |
| 1-19 | 1 mg | 12.0 mg | 0 mg | 16.0 mg | 2.0 mg |
| 1-20 | 1 mg | 12.0 mg | 0 mg | 11.0 mg | 2.0 mg |
| 1-21 | 1 mg | 12.0 mg | 0 mg | 7.0 mg | 1.0 mg |
| 1-22 | 1 mg | 12.0 mg | 0 mg | 5.0 mg | 1.0 mg |
| 1-23 | 1 mg | 12.0 mg | 0 mg | 3.0 mg | 1.0 mg |

| | | | | | |
|---|---|---|---|---|---|
| SM102: Heptadecan-9-yl 8-((2-hydroxyethyl) (6-oxo-6-(undecyloxy)hexyl)amino) octanoate (SINOPEG) DSPC: Distearoylphosphatidylcholine | | | | | |

### (3) Evaluation of physical properties of the formulation

1) For the prepared formulations, the particle characteristics of were confirmed using a dynamic light scattering (DLS) analyzer, and the results are shown in Table 3 below.
2) For the prepared formulations, the mRNA encapsulation efficiency was confirmed using a Ribo-green assay, and the results are shown in Table 3 below.

### (4) Administration of composition

The produced formulation was prepared at 10 µg/mL concentration, and administered intravenously to mice so that 2 µg based on mRNA was administered per mouse. After 4 hours, luciferin dissolved in sterile water was prepared at 15 µg/µL and administered intraperitoneally so that 3 mg of luciferin was administered per 20 g mouse. After 15 minutes from the intraperitoneal administration of luciferin, the results of protein expression for each organ were measured using a luminescence measurement imaging system and are shown in Table 3 below.

As confirmed in Table 3, the formulation for drug delivery according to the present invention had very excellent delivery efficiency to liver when administered intravenously.

Meanwhile, the formulation of Comparative Example 1 was intravenously administered to mice at the same amount of mRNA (2 µg/200 µL) as in the Examples, and the imaging results obtained after 4 hours using the same method as in the Examples are shown in Table 3. The Comparative Example formulation showed delivery to the liver upon intravenous administration.

**[Table 3]**

| | Zeta-average (nm) | PD index (PI) | Zeta-potential (mV) | Encapsulation efficiency (%) | Liver Avg Radiance [p/s/cm²/sr] |
|---|---|---|---|---|---|
| Comp. Example 1 | 105.8 ± 0.96 | 0.09 ± 0.02 | -3.24 ± 2.72 | 97.8 | 1.02E+08 |
| Example 1-1 | 151.8 ± 2.34 | 0.26 ± 0.04 | -6.59 ± 2.65 | 91.1 | 7.05E+07 |
| Example 1-2 | 158.0 ± 1.00 | 0.11 ± 0.02 | -6.08 ± 3.49 | 95.7 | 9.38E+07 |
| Example 1-3 | 154.8 ± 0.86 | 0.13 ± 0.01 | -6.47 ± 1.55 | 94.0 | 1.38E+08 |
| Example 1-4 | 160.2 ± 1.82 | 0.14 ± 0.01 | -4.59 ± 0.79 | 90.3 | 6.40E+07 |
| Example 1-5 | 156.6 ± 2.32 | 0.11 ± 0.02 | -4.80 ± 2.77 | 88.0 | 4.27E+07 |
| Example 1-6 | 162.4 ± 2.32 | 0.10 ± 0.01 | -5.88 ± 0.58 | 85.5 | 2.20E+07 |
| Example 1-7 | 140.4 ± 1.56 | 0.12 ± 0.01 | -2.55 ± 2.22 | 95.0 | 1.00E+08 |
| Example 1-8 | 136.9 ± 2.23 | 0.11 ± 0.00 | -5.62 ± 2.28 | 94.7 | 1.80E+08 |
| Example 1-9 | 152.7 ± 4.20 | 0.13 ± 0.02 | -2.37 ± 1.73 | 91.7 | 9.09E+07 |
| Example 1-10 | 169.8 ± 0.56 | 0.10 ± 0.01 | -5.10 ± 2.05 | 81.3 | 4.52E+07 |
| Example 1-11 | 166.2 ± 2.72 | 0.10 ± 0.02 | -4.20 ± 1.24 | 75.6 | 2.33E+07 |
| Example 1-12 | 147.9 ± 1.97 | 0.15 ± 0.01 | -6.00 ± 2.66 | 88.1 | 1.21E+08 |
| Example 1-13 | 152.1 ± 1.15 | 0.15 ± 0.00 | -8.20 ± 2.06 | 76.8 | 9.84E+07 |
| Example 1-14 | 171.2 ± 3.04 | 0.18 ± 0.02 | -9.71 ± 4.52 | 67.8 | 9.90E+06 |
| Example 1-15 | 152.5 ± 2.78 | 0.13 ± 0.01 | -14.22 ± 4.36 | 73.9 | 7.77E+07 |
| Example 1-16 | 133.5 ± 1.21 | 0.14 ± 0.01 | -13.19 ± 0.97 | 41.9 | 9.46E+07 |
| Example 1-17 | 177.0 ± 0.84 | 0.15 ± 0.01 | -13.29 ± 1.00 | 64.7 | 2.77E+07 |
| Example 1-18 | 142.2 ± 1.80 | 0.25 ± 0.03 | -14.11 ± 5.96 | 98.6 | 1.35E+07 |
| Example 1-19 | 169.1 ± 7.27 | 0.29 ± 0.04 | -15.43 ± 0.81 | 96.9 | 2.56E+07 |
| Example 1-20 | 126.9 ± 1.13 | 0.09 ± 0.02 | -7.70 ± 1.65 | 91.3 | 1.17E+07 |
| Example 1-21 | 177.6 ± 2.52 | 0.12 ± 0.04 | -2.94 ± 11.5 | 98.3 | 7.15E+07 |
| Example 1-22 | 161.1 ± 4.60 | 0.05 ± 0.05 | -11.4 ± 4.95 | 90.5 | 4.94E+07 |
| Example 1-23 | 180.1 ± 4.88 | 0.09 ± 0.02 | -6.33 ± 3.61 | 85.5 | 6.10E+07 |

### Example 2: Preparation of composition for drug delivery using the lipid of Preparation Example 1 and amphiphilic block copolymer, and test of drug delivery

### (1) Preparation of solutions for each component

By using the components shown in Table 4 below, solutions for each component were prepared in the same manner as the method of step (1) of Example 1 at the concentrations shown in Table 4 below.

**[Table 4]**

| No. | Components | Dilution solvents | Concentration for use |
|---|---|---|---|
| 1 | mRNA | RNAse Free water | 1 mg/mL |
| 2 | Lipid of Preparation Example 1 | Ethanol 100% | 10-20 mg/mL |
| 3 | DOPE | Ethanol 100% | 10-20 mg/mL |
| 4 | DOTAP(1,2-dioleoyl-3-trimethylammonium-propane) | Ethanol 100% | 10-20 mg/mL |
| 5 | Cholesterol | Ethanol 100% | 10-20 mg/mL |
| 6 | MPEG-PLA(2K-4K) | Ethanol 95% | 50-100 mg/mL |

| | | | |
|---|---|---|---|
| MPEG-PLA(2K-4K): Copolymer of a monomethoxypolyethylene glycol (mPEG) block with a number average molecular weight of 2,000 and a polylactic acid (PLA) block with a number average molecular weight of 4,000 | | | |

### (2) Mixing of raw materials

The required amounts of the components were taken in order to meet the N/P ratio (amine group of lipid component/phosphate group of mRNA) of 6 and the ratio of Lipid of Preparation Example 1:DOPE or DOTAP:cholesterol:MPEG-PLA(2K-4K) as shown in Table 5 below, and mixed in the same manner as the method of step (2) of Example 1. (The exception is that under Micofluidics operating conditions, the Total Flow Rate (TRR) was 3 mL/min.)

**[Table 5]**

| | Effective ingredient | Lipid | Fusogenic lipid | | Polymer |
|---|---|---|---|---|---|
| Comp. Example | mRNA | SM102 | DSPC | Cholesterol | DMG-PEG |
| 1 | 1 mg | 11.4 mg | 2.5 mg | 4.8 mg | 1.2 mg |
| Example | mRNA | Lipid of Prep. Ex. 1 | DOPE | Cholesterol | MPEG-PLA(2K-4K) |
| 2-1 | 1 mg | 12.0 mg | 2.0 mg | 2.1 mg | 33.3 mg |
| 2-2 | 1 mg | 12.0 mg | 4.0 mg | 1.0 mg | 33.3 mg |
| 2-3 | 1 mg | 12.0 mg | 2.3 mg | 3.6 mg | 38.9 mg |
| 2-4 | 1 mg | 12.0 mg | 4.6 mg | 2.4 mg | 38.9 mg |
| 2-5 | 1 mg | 12.0 mg | 6.9 mg | 1.2 mg | 38.9 mg |
| 2-6 | 1 mg | 12.0 mg | 2.8 mg | 5.7 mg | 46.6 mg |
| 2-7 | 1 mg | 12.0 mg | 5.5 mg | 4.3 mg | 46.6 mg |
| 2-8 | 1 mg | 12.0 mg | 8.3 mg | 2.9 mg | 46.6 mg |
| 2-9 | 1 mg | 12.0 mg | 11.1 mg | 1.4 mg | 46.6 mg |
| 2-10 | 1 mg | 12.0 mg | 13.8 mg | 3.6 mg | 58.3 mg |
| 2-11 | 1 mg | 12.0 mg | 6.9 mg | 7.2 mg | 58.3 mg |
| 2-12 | 1 mg | 12.0 mg | 1.7 mg | 0.9 mg | 29.1 mg |
| 2-13 | 1 mg | 24.1 mg | 9.2 mg | 4.8 mg | 77.7 mg |
| 2-14 | 1 mg | 22.9 mg | 3.8 mg | 3.9 mg | 151.5 mg |
| 2-15 | 1 mg | 22.9 mg | 7.5 mg | 2.0 mg | 75.8 mg |
| 2-16 | 1 mg | 22.9 mg | 7.5 mg | 2.0 mg | 151.5 mg |
| 2-17 | 1 mg | 22.9 mg | 7.5 mg | 2.0 mg | 303.0 mg |
| 2-18 | 1 mg | 22.9 mg | 4.4 mg | 6.8 mg | 176.8 mg |
| 2-19 | 1 mg | 22.9 mg | 8.8 mg | 4.6 mg | 88.4 mg |
| 2-20 | 1 mg | 22.9 mg | 8.8 mg | 4.6 mg | 176.8 mg |
| 2-21 | 1 mg | 22.9 mg | 8.8 mg | 4.6 mg | 353.5 mg |
| 2-22 | 1 mg | 22.9 mg | 13.2 mg | 2.3 mg | 88.4 mg |
| 2-23 | 1 mg | 17.2 mg | 9.9 mg | 1.7 mg | 132.6 mg |
| 2-24 | 1 mg | 22.9 mg | 13.2 mg | 2.3 mg | 176.8 mg |
| 2-25 | 1 mg | 22.9 mg | 13.2 mg | 2.3 mg | 353.5 mg |
| 2-26 | 1 mg | 17.2 mg | 7.9 mg | 6.2 mg | 159.1 mg |
| 2-27 | 1 mg | 22.9 mg | 10.5 mg | 8.2 mg | 212.1 mg |
| 2-28 | 1 mg | 17.2 mg | 11.8 mg | 4.1 mg | 159.1 mg |
| 2-29 | 1 mg | 22.9 mg | 15.8 mg | 5.5 mg | 212.1 mg |
| Example | mRNA | Lipid of Prep. Ex. 1 | DOTAP | Cholesterol | MPEG-PLA(2K-4K) |
| 2-30 | 1 mg | 22.9 mg | 3.3 mg | 3.9 mg | 151.5 mg |
| 2-31 | 1 mg | 22.9 mg | 6.7 mg | 2.0 mg | 151.5 mg |
| 2-32 | 1 mg | 22.9 mg | 3.9 mg | 6.8 mg | 176.8 mg |
| 2-32 | 1 mg | 22.9 mg | 7.8 mg | 4.6 mg | 88.4 mg |
| 2-33 | 1 mg | 11.5 mg | 3.9 mg | 2.3 mg | 88.4 mg |
| 2-34 | 1 mg | 17.2 mg | 5.9 mg | 3.4 mg | 132.6 mg |
| 2-35 | 1 mg | 22.9 mg | 7.8 mg | 4.6 mg | 176.8 mg |
| 2-36 | 1 mg | 22.9 mg | 7.8 mg | 4.6 mg | 353.5 mg |
| 2-37 | 1 mg | 22.9 mg | 11.7 mg | 2.3 mg | 176.8 mg |
| 2-38 | 1 mg | 22.9 mg | 9.4 mg | 8.2 mg | 106.1 mg |
| 2-39 | 1 mg | 11.5 mg | 4.7 mg | 4.1 mg | 106.1 mg |
| 2-40 | 1 mg | 17.2 mg | 7.0 mg | 6.2 mg | 159.1 mg |
| 2-41 | 1 mg | 22.9 mg | 9.4 mg | 8.2 mg | 212.1 mg |
| 2-42 | 1 mg | 22.9 mg | 9.4 mg | 8.2 mg | 424.2 mg |
| 2-43 | 1 mg | 22.9 mg | 14.1 mg | 5.5 mg | 106.1 mg |
| 2-44 | 1 mg | 22.9 mg | 14.1 mg | 5.5 mg | 212.1 mg |
| 2-45 | 1 mg | 22.9 mg | 14.1 mg | 5.5 mg | 424.2 mg |
| Example | mRNA | Lipid of Prep. Ex. 1 | DOTAP | Cholesterol | MPEG-PLA(2K-3K) |
| 2-46 | 1 mg | 24.1 mg | 8.7 mg | 4.8 mg | 154.9 mg |
| 2-47 | 1 mg | 24.1 mg | 8.7 mg | 4.8 mg | 185.9 mg |
| Example | mRNA | Lipid of Prep. Ex. 1 | DOTAP | Cholesterol | MPEG-PLA(2K-5.4K) |
| 2-48 | 1 mg | 24.1 mg | 8.7 mg | 4.8mg | 185.9 mg |

### (3) Evaluation of physical properties of the formulation

For the prepared formulations, the physical properties were evaluated in the same manner as the method of step (3) of Example 1, and the results are shown in Table 6 below.

**[Table 6]**

| | Zeta-average (nm) | PD index (PI) | Zeta-potential (mV) | Encapsulation efficiency (%) |
|---|---|---|---|---|
| Comp. Example 1 | 105.8 ± 0.96 | 0.09 ± 0.02 | -3.24 ± 2.72 | 97.8 |
| Example 2-1 | 124.7 ± 1.40 | 0.16 ± 0.02 | -20.9 ± 5.84 | 74.2 |
| Example 2-2 | 160.1 ± 2.83 | 0.10 ± 0.03 | -14.4 ± 5.60 | 83.9 |
| Example 2-3 | 139.1 ± 0.91 | 0.10 ± 0.02 | -5.6 ± 0.90 | 86.4 |
| Example 2-4 | 144.3 ± 1.50 | 0.09 ± 0.00 | -7.2 ± 5.44 | 65.6 |
| Example 2-5 | 139.8 ± 1.41 | 0.08 ± 0.02 | -5.9 ± 0.47 | 94.5 |
| Example 2-6 | 152.5 ± 1.27 | 0.14 ± 0.01 | -6.3 ± 5.14 | 91.1 |
| Example 2-7 | 137.3 ± 1.70 | 0.12 ± 0.03 | -5.0 ± 0.78 | 96.9 |
| Example 2-8 | 142.3 ± 0.46 | 0.07 ± 0.01 | -2.6 ± 2.12 | 96.9 |
| Example 2-9 | 147.7 ± 0.66 | 0.06 ± 0.02 | -8.5 ± 1.60 | 97.4 |
| Example 2-10 | 220.0 ± 4.71 | 0.13 ± 0.02 | -17.5 ± 7.70 | 96.4 |
| Example 2-11 | 174.1 ± 6.64 | 0.11 ± 0.02 | -18.7 ± 4.61 | 93.7 |
| Example 2-12 | 153.5 ± 0.20 | 0.14 ± 0.01 | -15.9 ± 2.13 | 79.6 |
| Example 2-13 | 141.3 ± 0.20 | 0.08 ± 0.02 | -9.82 ± 1.78 | 82.7 |
| Example 2-14 | 129.0 ± 1.47 | 0.10 ± 0.01 | -8.60 ± 0.48 | 66.7 |
| Example 2-15 | 180.2 ± 2.25 | 0.05 ± 0.02 | -5.64 ± 0.19 | 78.5 |
| Example 2-16 | 136.6 ± 0.92 | 0.08 ± 0.02 | -7.81 ± 0.54 | 77.5 |
| Example 2-17 | 120.6 ± 1.04 | 0.14 ± 0.01 | -7.80 ± 0.34 | 78.7 |
| Example 2-18 | 149.1 ± 1.30 | 0.13 ± 0.02 | -3.97 ± 0.69 | 79.3 |
| Example 2-19 | 181.8 ± 0.74 | 0.06 ± 0.02 | -10.66 ± 1.46 | 86.4 |
| Example 2-20 | 151.6 ± 2.66 | 0.10 ± 0.06 | -9.08 ± 0.80 | 86.7 |
| Example 2-21 | 127.0 ± 6.87 | 0.18 ± 0.07 | -7.16 ± 0.43 | 85.5 |
| Example 2-22 | 205.4 ± 2.26 | 0.05 ± 0.02 | -6.81 ± 0.13 | 93.5 |
| Example 2-23 | 152.6 ± 0.94 | 0.07 ± 0.01 | -4.38 ± 0.89 | 78.3 |
| Example 2-24 | 153.0 ± 1.39 | 0.08 ± 0.02 | -1.91 ± 1.69 | 92.3 |
| Example 2-25 | 115.3 ± 0.54 | 0.12 ± 0.02 | -5.60 ± 0.55 | 85.5 |
| Example 2-26 | 162.5 ± 2.14 | 0.08 ± 0.01 | -1.64 ± 0.79 | 85.7 |
| Example 2-27 | 156.3 ± 1.06 | 0.08 ± 0.02 | -4.73 ± 2.08 | 80.9 |
| Example 2-28 | 161.9 ± 0.52 | 0.07 ± 0.00 | -3.66 ± 2.12 | 85.7 |
| Example 2-29 | 163.7 ± 1.30 | 0.07 ± 0.02 | -1.65 ± 2.72 | 82.2 |
| Example 2-30 | 120.4 ± 0.67 | 0.10 ± 0.01 | 7.95 ± 1.49 | 79.2 |
| Example 2-31 | 145.0 ± 0.93 | 0.09 ± 0.03 | -0.60 ± 1.42 | 97.5 |
| Example 2-32 | 173.1 ± 1.38 | 0.19 ± 0.01 | -4.12 ± 4.75 | 91.3 |
| Example 2-32 | 179.6 ± 0.79 | 0.14 ± 0.01 | 1.41 ± 3.81 | 98.7 |
| Example 2-33 | 234.7 ± 4.51 | 0.14 ± 0.00 | -6.46 ± 2.71 | 95.8 |
| Example 2-34 | 221.6 ± 12.98 | 0.22 ± 0.03 | -0.42 ± 2.09 | 96.4 |
| Example 2-35 | 130.4 ± 0.09 | 0.10 ± 0.01 | 3.34 ± 1.27 | 97.8 |
| Example 2-36 | 126.5 ± 0.87 | 0.18 ± 0.02 | 2.53 ± 0.71 | 97.4 |
| Example 2-37 | 100.4 ± 0.96 | 0.12 ± 0.02 | 6.87 ± 3.07 | 97.7 |
| Example 2-38 | 161.7 ± 2.49 | 0.13 ± 0.03 | 10.92 ± 0.67 | 94.1 |
| Example 2-39 | 220.6 ± 5.72 | 0.18 ± 0.01 | -13.21 ± 2.39 | 94.5 |
| Example 2-40 | 163.0 ± 3.74 | 0.17 ± 0.01 | -2.85 ± 3.45 | 96.2 |
| Example 2-41 | 124.1 ± 2.25 | 0.13 ± 0.01 | 5.81 ± 1.66 | 98.7 |
| Example 2-42 | 102.5 ± 1.52 | 0.20 ± 0.01 | 1.92 ± 1.13 | 97.3 |
| Example 2-43 | 118.6 ± 1.60 | 0.14 ± 0.00 | 7.80 ± 1.75 | 97.6 |
| Example 2-44 | 87.6 ± 0.22 | 0.17 ± 0.01 | 8.53 ± 1.77 | 99.5 |
| Example 2-45 | 85.4 ± 1.32 | 0.21 ± 0.01 | 7.60 ± 1.35 | 97.3 |
| Example 2-46 | 130.6 ± 1.19 | 0.09 ± 0.01 | 12.06 ± 0.65 | 97.0 |
| Example 2-47 | 133.0 ± 0.76 | 0.11 ± 0.02 | 10.29 ± 2.40 | 96.5 |
| Example 2-48 | 90.8 ± 0.92 | 0.15 ± 0.02 | 0.91 ± 0.35 | 95.7 |

### (4) Administration of composition

The produced formulation was prepared at 10 µg/mL concentration, and administered to mice with 2 µg based on mRNA per mouse in case of intravascular (IV) administration, 2.5 µg based on mRNA per mouse in case of intratracheal (IT) injection, and 2 µg based on mRNA per mouse in case of intramuscular (IM) administration. After each administration, luciferin dissolved in sterile water was prepared at 15 µg/µL and administered intraperitoneally so that 3 mg of luciferin was administered per 20 g mouse. After 15 minutes from the intraperitoneal administration of luciferin, the results of protein expression for each organ were measured using a luminescence measurement imaging system and are shown in Tables 7, 8 and 9 below.

As confirmed in Tables 7, 8 and 9, the formulation for drug delivery according to the present invention had very excellent delivery efficiency to the target organ upon each administration.

Meanwhile, the formulation of Comparative Example 1 was intravenously administered to mice at the same amount of mRNA (2 µg/200 µL) as in the Examples, and the imaging results obtained at the time of 4 hours after intravenous administration, 6 hours after intratracheal injection, and 4 and 24 hours after intramuscular administration, using the same method as in the Examples are shown in Tables 7, 8 and 9. As confirmed in Tables 7, 8 and 9, the Comparative Example formulation showed delivery to the target organ upon each administration.

**[Table 7]**

| IV administration | Liver Avg Radiance[p/s/cm²/sr] |
|---|---|
| Comp. Example 1 | 1.02E+08 |
| Example 2-1 | 1.36E+07 |
| Example 2-2 | 5.44E+06 |
| Example 2-3 | 8.14E+07 |
| Example 2-4 | 5.64E+07 |
| Example 2-5 | 4.31E+07 |
| Example 2-6 | 3.51E+07 |
| Example 2-7 | 9.60E+07 |
| Example 2-8 | 7.26E+07 |
| Example 2-9 | 7.06E+07 |
| Example 2-10 | 2.86E+07 |
| Example 2-11 | 3.15E+07 |
| Example 2-12 | 1.67E+05 |

**[Table 8]**

| IT administration | Lung Avg Radiance[p/s/cm²/sr] |
|---|---|
| Comp. Example 1 | 1.79E+05 |
| Example 2-1 | 1.65E+05 |
| Example 2-2 | 6.15E+05 |
| Example 2-3 | 2.09E+05 |
| Example 2-4 | 4.84E+05 |
| Example 2-5 | 1.89E+05 |
| Example 2-6 | 4.95E+05 |
| Example 2-7 | 3.93E+05 |
| Example 2-8 | 3.24E+05 |
| Example 2-9 | 2.08E+05 |
| Example 2-12 | 4.10E+03 |
| Example 2-13 | 1.42E+06 |
| Example 2-14 | 1.94E+06 |
| Example 2-15 | 1.94E+06 |
| Example 2-16 | 1.74E+06 |
| Example 2-17 | 4.62E+06 |
| Example 2-18 | 5.19E+05 |
| Example 2-19 | 2.22E+06 |
| Example 2-20 | 3.23E+06 |
| Example 2-21 | 8.55E+06 |
| Example 2-22 | 2.00E+06 |
| Example 2-23 | 4.40E+06 |
| Example 2-24 | 3.94E+06 |
| Example 2-25 | 2.07E+06 |
| Example 2-26 | 2.04E+06 |
| Example 2-27 | 2.71E+06 |
| Example 2-28 | 2.93E+06 |
| Example 2-29 | 3.28E+06 |
| Example 2-30 | 9.38E+05 |
| Example 2-31 | 3.41E+06 |
| Example 2-32 | 6.67E+06 |
| Example 2-32 | 3.97E+06 |
| Example 2-33 | 4.67E+06 |
| Example 2-34 | 5.28E+05 |
| Example 2-35 | 1.21E+07 |
| Example 2-36 | 3.98E+06 |
| Example 2-37 | 3.87E+06 |
| Example 2-38 | 7.55E+06 |
| Example 2-39 | 2.12E+06 |
| Example 2-40 | 9.64E+05 |
| Example 2-41 | 1.44E+07 |
| Example 2-42 | 8.22E+06 |
| Example 2-43 | 7.64E+06 |
| Example 2-44 | 8.17E+06 |
| Example 2-45 | 6.41E+06 |
| Example 2-46 | 2.31E+06 |
| Example 2-47 | 2.08E+06 |
| Example 2-48 | 3.50E+07 |

**[Table 9]**

| IM administration | Injection point Avg Radiance[p/s/cm²/sr] | |
|---|---|---|
| | 4 hr | 24 hr |
| Comp. Example 1 | 1.72E+07 | 4.46E+05 |
| Example 2-1 | 8.19E+06 | 5.33E+05 |
| Example 2-2 | 2.75E+06 | 4.25E+05 |
| Example 2-3 | 3.31E+07 | 1.93E+06 |
| Example 2-4 | 2.80E+07 | 2.54E+06 |
| Example 2-5 | 4.29E+07 | 5.44E+06 |
| Example 2-6 | 2.20E+07 | 1.94E+06 |
| Example 2-7 | 5.66E+07 | 1.94E+06 |
| Example 2-8 | 5.82E+07 | 8.21E+06 |
| Example 2-9 | 6.12E+07 | 5.29E+06 |
| Example 2-10 | 6.18E+05 | 3.02E+05 |
| Example 2-11 | 2.89E+06 | 6.64E+05 |
| Example 2-12 | 3.05E+06 | 6.87E+05 |

### Example 3: Preparation of composition for drug delivery using the lipid of Preparation Example 5 and amphiphilic block copolymer, and test of drug delivery

### (1) Preparation of solutions for each component

By using the components shown in Table 10 below, solutions for each component were prepared in the same manner as the method of step (1) of Example 1 at the concentrations shown in Table 10 below.

**[Table 10]**

| No. | Components | Dilution solvents | Concentration for use |
|---|---|---|---|
| 1 | mRNA | RNAse Free water | 1 mg/mL |
| 2 | Lipid of Preparation Example 5 | Ethanol 100% | 10-20 mg/mL |
| 3 | DOPE | Ethanol 100% | 10-20 mg/mL |
| 4 | DOTAP | Ethanol 100% | 10-20 mg/mL |
| 5 | Cholesterol | Ethanol 100% | 10-20 mg/mL |
| 6 | MPEG-PLA(2K-4K) | Ethanol 95% | 50-100 mg/mL |

### (2) Mixing of raw materials

The required amounts of the components were taken in order to meet the N/P ratio (amine group of lipid component/phosphate group of mRNA) of 6 and the ratio of Lipid of Preparation Example 5:DOPE or DOTAP:cholesterol:MPEG-PLA(2K-4K) as shown in Table 11 below, and mixed in the same manner as the method of step (2) of Example 1.

**[Table 11]**

| | Effective ingredient | Lipid | Fusogenic lipid | | Polymer |
|---|---|---|---|---|---|
| Example | mRNA | Lipid of Prep. Ex. 5 | DOPE | Cholesterol | MPEG-PLA(2K-4K) |
| 3-1 | 1 mg | 12.2 mg | 0 mg | 0.8 mg | 60 mg |
| 3-2 | 1 mg | 12.2 mg | 1.7 mg | 0.9 mg | 68 mg |
| 3-3 | 1 mg | 12.2 mg | 3.8 mg | 1 mg | 77 mg |
| 3-4 | 1 mg | 12.2 mg | 10.7 mg | 1.4 mg | 108 mg |
| 3-5 | 1 mg | 12.2 mg | 0 mg | 1.7 mg | 67.6 mg |
| 3-6 | 1 mg | 12.2 mg | 1.9 mg | 2 mg | 77 mg |
| 3-7 | 1 mg | 12.2 mg | 4.5 mg | 2.3 mg | 90 mg |
| 3-8 | 1 mg | 12.2 mg | 13.4 mg | 3.5 mg | 135 mg |
| 3-9 | 1 mg | 12.2 mg | 2.7 mg | 5.6 mg | 108 mg |
| 3-10 | 1 mg | 12.2 mg | 6.7 mg | 7.0 mg | 135 mg |
| 3-11 | 1 mg | 12.2 mg | 4.9 mg | 3.2 mg | 98 mg |
| 3-12 | 1 mg | 12.2 mg | 5.4 mg | 4.2 mg | 108 mg |
| 3-13 | 1 mg | 12.2 mg | 2.7 mg | 5.6 mg | 54 mg |
| 3-14 | 1 mg | 12.2 mg | 2.7 mg | 5.6 mg | 162 mg |
| 3-15 | 1 mg | 6.1 mg | 1.3 mg | 2.8 mg | 54 mg |
| 3-16 | 1 mg | 18.3 mg | 4 mg | 8.4 mg | 162 mg |
| 3-17 | 1 mg | 12.0 mg | 2.6 mg | 5.5 mg | 106.1 mg |
| 3-18 | 1 mg | 24.0 mg | 8.8 mg | 4.6 mg | 176.8 mg |
| 3-19 | 1 mg | 12.0 mg | 2.6 mg | 5.4 mg | 1.4 mg |
| Example | mRNA | Lipid of Prep. Ex. 5 | DOTAP | Cholesterol | MPEG-PLA(2K-4K) |
| 3-20 | 1 mg | 24.0 mg | 7.8 mg | 4.6 mg | 176.8 mg |
| 3-21 | 1 mg | 12.6 mg | 4.3 mg | 2.4 mg | 92.9 mg |
| 3-22 | 1 mg | 18.9 mg | 6.5 mg | 3.6 mg | 139.4 mg |
| 3-23 | 1 mg | 12.6 mg | 5.2 mg | 4.3 mg | 111.5 mg |
| 3-24 | 1 mg | 18.9 mg | 7.8 mg | 6.5 mg | 167.3 mg |
| 3-25 | 1 mg | 25.2 mg | 10.4 mg | 8.6 mg | 222 mg |
| 3-26 | 1 mg | 25.2 mg | 15.6 mg | 5.7 mg | 223 mg |
| Example | mRNA | Lipid of Prep. Ex. 5 | DOTAP | Cholesterol | MPEG-PLA(2K-3K) |
| 3-27 | 1 mg | 25.2 mg | 8.7 mg | 4.8 mg | 154.9 mg |
| 3-28 | 1 mg | 25.2 mg | 8.7 mg | 4.8 mg | 185.9 mg |
| Example | mRNA | Lipid of Prep. Ex. 5 | DPPC | Cholesterol | MPEG-PLA(2K-4K) |
| 3-29 | 1 mg | 25.2 mg | 9.1 mg | 4.8 mg | 185.9 mg |

### (3) Evaluation of physical properties of the formulation

For the prepared formulations, the physical properties were evaluated in the same manner as the method of step (3) of Example 1, and the results are shown in Table 12 below.

**[Table 12]**

| | Zeta-average (nm) | PD index (PI) | Zeta-potential (mV) | Encapsulation efficiency (%) |
|---|---|---|---|---|
| Example 3-1 | 110.2 ± 0.72 | 0.198 ± 0.002 | -15.2 ± 1.313 | 88.9 |
| Example 3-2 | 116.9 ± 0.35 | 0.134 ± 0.014 | -1.5 ± 0.94 | 89.5 |
| Example 3-3 | 166.2 ± 0.78 | 0.179 ± 0.021 | -8.8 ± 0.342 | 87.5 |
| Example 3-4 | 182.5 ± 1.1 | 0.097 ± 0.042 | -1.3 ± 0.503 | 90.8 |
| Example 3-5 | 105.6 ± 0.6 | 0.177 ± 0.015 | -12.8 ± 0.804 | 94.0 |
| Example 3-6 | 126.6 ± 1.12 | 0.112 ± 0.03 | -8.8 ± 2.546 | 84.2 |
| Example 3-7 | 103.3 ± 0.4 | 0.162 ± 0.023 | -5.4 ± 0.653 | 94.8 |
| Example 3-8 | 208.7±4.27 | 0.134 ± 0.024 | -8.8 ± 1.08 | 72.5 |
| Example 3-9 | 135.5 ± 1.7 | 0.094 ± 0.004 | -4.8 ± 0.321 | 97.4 |
| Example 3-10 | 180.2 ± 2.76 | 0.128 ± 0.006 | -3.8 ± 0.554 | 95.1 |
| Example 3-11 | 132.4 ± 0.35 | 0.124 ± 0.040 | -5.2 ± 0.346 | 92.1 |
| Example 3-12 | 117.3 ± 2.08 | 0.113 ± 0.033 | -6.3 ± 0.706 | 35.1 |
| Example 3-13 | 188.7 ± 1.24 | 0.053 ± 0.007 | -9.7 ± 1.554 | 87.1 |
| Example 3-14 | 117.7 ± 2.83 | 0.157 ± 0.013 | -5.5 ± 0.477 | 89.3 |
| Example 3-15 | 144.3 ± 1.35 | 0.130 ± 0.011 | -10.9 ± 0.964 | 85.4 |
| Example 3-16 | 147.3 ± 2.37 | 0.143 ± 0.011 | -5.7 ± 2.415 | 90.1 |
| Example 3-17 | 113.0 ± 0.71 | 0.07 ± 0.02 | -6.82 ± 0.75 | 94.2 |
| Example 3-18 | 243.0 ± 5.69 | 0.21 ± 0.02 | -0.40 ± 1.80 | 91.8 |
| Example 3-19 | 149.3 ± 1.99 | 0.095 ± 0.034 | -8.3±2.912 | 81.4 |
| Example 3-20 | 114.7 ± 1.37 | 0.14 ± 0.01 | 12.47 ± 1.76 | 94.7 |
| Example 3-21 | 131.5 ± 1.65 | 0.08 ± 0.02 | -4.15 ± 2.81 | 80.1 |
| Example 3-22 | 120.6 ± 1.51 | 0.10 ± 0.00 | -0.26 ± 1.29 | 94.8 |
| Example 3-23 | 161.9 ± 2.34 | 0.07 ± 0.01 | -3.81 ± 0.91 | 94.0 |
| Example 3-24 | 107.4 ± 1.40 | 0.13 ± 0.01 | -1.64 ± 2.53 | 95.3 |
| Example 3-25 | 122.7 ± 1.55 | 0.18 ± 0.02 | -5.36 ± 0.66 | 95.3 |
| Example 3-26 | 111.3 ± 4.16 | 0.20 ± 0.01 | -3.4 ± 2.31 | 94.7 |
| Example 3-27 | 168.5 ± 1.76 | 0.04 ± 0.02 | 6.20 ± 1.42 | 96.9 |
| Example 3-28 | 132.4 ± 0.74 | 0.07 ± 0.01 | 10.85 ± 1.44 | 96.9 |
| Example 3-29 | 104.9 ± 0.74 | 0.15 ± 0.01 | -2.80 ± 0.80 | 69.1 |

### (4) Administration of composition

The produced formulation was prepared at 10 µg/mL concentration, and administered to mice with 2 µg based on mRNA per mouse in case of intravascular (IV) administration, and 2.5 µg based on mRNA per mouse in case of intratracheal (IT) injection. After each administration, luciferin dissolved in sterile water was prepared at 15 µg/µL and administered intraperitoneally so that 3 mg of luciferin was administered per 20 g mouse. After 15 minutes from the intraperitoneal administration of luciferin, the results of protein expression for each organ were measured using a luminescence measurement imaging system and are shown in Tables 13 and 14 below.

As confirmed in Tables 13 and 14, the formulation for drug delivery according to the present invention had very excellent delivery efficiency to the target organ upon each administration.

**[Table 13]**

| IV administration | Liver Avg Radiance[p/s/cm²/sr] |
|---|---|
| Example 3-1 | 2.2.E+03 |
| Example 3-2 | 2.0.E+07 |
| Example 3-3 | 4.7.E+07 |
| Example 3-4 | 7.6.E+07 |
| Example 3-5 | 1.0.E+06 |
| Example 3-6 | 2.8.E+08 |
| Example 3-7 | 3.2.E+08 |
| Example 3-8 | 2.8.E+08 |
| Example 3-9 | 6.6.E+08 |
| Example 3-10 | 3.5.E+08 |
| Example 3-11 | 5.1.E+08 |
| Example 3-12 | 6.1.E+08 |
| Example 3-13 | 2.0.E+08 |
| Example 3-14 | 2.2.E+08 |
| Example 3-15 | 1.9.E+08 |
| Example 3-16 | 5.2.E+08 |
| Example 3-19 | 5.8E+08 |

**[Table 14]**

| IT administration | Lung Avg Radiance[p/s/cm²/sr] |
|---|---|
| Example 3-17 | 3.78E+06 |
| Example 3-18 | 3.19E+06 |
| Example 3-20 | 1.12E+07 |

### Example 4: Preparation of composition for drug delivery using the lipid of Preparation Example 6 and amphiphilic block copolymer, and test of drug delivery

### (1) Preparation of solutions for each component

By using the components shown in Table 15 below, solutions for each component were prepared in the same manner as the method of step (1) of Example 1 at the concentrations shown in Table 15 below.

**[Table 15]**

| No. | Components | Dilution solvents | Concentration for use |
|---|---|---|---|
| 1 | mRNA | RNAse Free water | 1 mg/mL |
| 2 | Lipid of Preparation Example 6 | Ethanol 100% | 10-20 mg/mL |
| 3 | DOPE | Ethanol 100% | 10-20 mg/mL |
| 4 | DOTAP | Ethanol 100% | 10-20 mg/mL |
| 5 | Cholesterol | Ethanol 100% | 10-20 mg/mL |
| 6 | MPEG-PLA(2K-4K) | Ethanol 95% | 50-100 mg/mL |

### (2) Mixing of raw materials

The required amounts of the components were taken in order to meet the N/P ratio (amine group of lipid component/phosphate group of mRNA) of 6 and the ratio of Lipid of Preparation Example 6:DOPE or DOTAP:cholesterol:MPEG-PLA(2K-4K) as shown in Table 16 below, and mixed in the same manner as the method of step (2) of Example 1.

**[Table 16]**

| | Effective ingredient | Lipid | Fusogenic lipid | | Polymer |
|---|---|---|---|---|---|
| Example | mRNA | Lipid of Prep. Ex. 6 | DOPE | Cholesterol | MPEG-PLA(2K-4K) |
| 4-1 | 1 mg | 12.0 mg | 10.4 mg | 5.4 mg | 139.4 mg |
| 4-2 | 1 mg | 12.0 mg | 6.9 mg | 7.2 mg | 139.4 mg |
| 4-3 | 1 mg | 12.0 mg | 2.8 mg | 5.7 mg | 111.5 mg |
| 4-4 | 1 mg | 12.0 mg | 5.5 mg | 4.3 mg | 111.5 mg |
| 4-5 | 1 mg | 12.0 mg | 8.3 mg | 2.9 mg | 111.5 mg |
| 4-6 | 1 mg | 12.0 mg | 2.3 mg | 3.6 mg | 92.9 mg |
| 4-7 | 1 mg | 12.0 mg | 4.6 mg | 2.4 mg | 92.9 mg |
| 4-8 | 1 mg | 12.0 mg | 6.9 mg | 1.2 mg | 92.9 mg |
| 4-9 | 1 mg | 12.0 mg | 2.8 mg | 5.5 mg | 1.4 mg |

| Example | mRNA | Lipid of Prep. Ex. 6 | DOTAP | Cholesterol | MPEG-PLA(2K-4K) |
|---|---|---|---|---|---|
| 4-10 | 1 mg | 24.0 mg | 7.8 mg | 4.6 mg | 176.8 mg |

### (3) Evaluation of physical properties of the formulation

For the prepared formulations, the physical properties were evaluated in the same manner as the method of step (3) of Example 1, and the results are shown in Table 17 below.

**[Table 17]**

| | Zeta-average (nm) | PD index (PI) | Zeta-potential (mV) | Encapsulation efficiency (%) |
|---|---|---|---|---|
| Example 4-1 | 132.4 ± 0.4 | 0.070 ± 0.017 | -8.2 ± 1.2 | 97.8 |
| Example 4-2 | 107.5 ± 0.5 | 0.117 ± 0.003 | -5.8 ± 0.6 | 97.5 |
| Example 4-3 | 158.3 ± 1.8 | 0.093 ± 0.026 | -5.3 ± 4.1 | 97.7 |
| Example 4-4 | 122.4 ± 2.0 | 0.067 ± 0.004 | -5.5 ± 0.3 | 97.3 |
| Example 4-5 | 156.0 ± 0.4 | 0.063 ± 0.042 | -5.3 ± 2.9 | 98.4 |
| Example 4-6 | 101.3 ± 2.0 | 0.131 ± 0.017 | -5.9 ± 0.4 | 72.7 |
| Example 4-7 | 161.8 ± 2.1 | 0.141 ± 0.017 | -2.5 ± 2.4 | 68.4 |
| Example 4-8 | 92.7 ± 1.2 | 0.124 ± 0.012 | -3.7 ± 3.0 | 39.3 |
| Example 4-9 | 98.0 ± 1.5 | 0.118 ± 0.028 | -5.1 ± 0.9 | 90.6 |
| Example 4-10 | 142.0 ± 2.20 | 0.06 ± 0.03 | -0.7 ± 2.2 | 97.7 |

### (4) Administration of composition

The produced formulation was prepared at 10 µg/mL concentration, and administered to mice with 2 µg based on mRNA per mouse in case of intravascular (IV) administration, and 2.5 µg based on mRNA per mouse in case of intratracheal (IT) injection. After each administration, luciferin dissolved in sterile water was prepared at 15 µg/µL and administered intraperitoneally so that 3 mg of luciferin was administered per 20 g mouse. After 15 minutes from the intraperitoneal administration of luciferin, the results of protein expression for each organ were measured using a luminescence measurement imaging system and are shown in Tables 18 and 19 below.

As confirmed in Tables 18 and 19, the formulation for drug delivery according to the present invention had very excellent delivery efficiency to the target organ upon each administration.

**[Table 18]**

| IV administration | Liver Avg Radiance[p/s/cm²/sr] |
|---|---|
| Example 4-1 | 1.48E+08 |
| Example 4-2 | 8.67E+07 |
| Example 4-3 | 3.49E+07 |
| Example 4-4 | 1.81E+08 |
| Example 4-5 | 5.51E+07 |
| Example 4-6 | 1.53E+08 |
| Example 4-7 | 3.62E+07 |
| Example 4-8 | 1.88E+07 |
| Example 4-9 | 1.17E+08 |

**[Table 19]**

| IT administration | Lung Avg Radiance[p/s/cm²/sr] |
|---|---|
| Example 4-10 | 3.66E+06 |

## Claims

1. A composition for drug delivery comprising:
effective ingredient selected from nucleic acid, polypeptide, virus or combination thereof;
a lipid having a structure represented by the following formula 1; and
lipid-polymer, amphiphilic block copolymer, or a combination thereof: 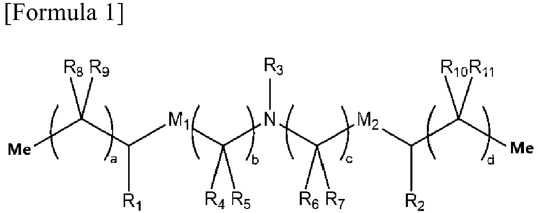
wherein, in the above formula 1,
each of M₁ and M₂ is independently a divalent linker group,
each of R₁ and R₂ is independently a substituted or unsubstituted carbocyclic group or heterocyclic group,
R₃ is hydrogen atom, or a substituted or unsubstituted organic group optionally comprising one or more heteroatoms,
each of R₄ to R₁₁ is independently hydrogen atom, or a substituted or unsubstituted, saturated or unsaturated hydrocarbon group,
Me is methyl group, and
each of a, b, c and d is independently an integer of from 1 to 20.

2. The composition for drug delivery according to claim 1, wherein
each of M₁ and M₂ is independently selected from the group consisting of -C(O)O-, - OC(O)-, -OC(O)-M'-C(O)O-, -C(O)N(R')-, -N(R')C(O)-, -C(O)-, -C(S)-, -C(S)S-, -SC(S)-, - CH(OH)-, -P(O)(OR')O-, -S(O)₂-, -S-S-, arylene, and heteroarylene, wherein M' is a direct bond, C₁₋₁₃ alkylene or C₂₋₁₃ alkenylene, and each R' is independently selected from the group consisting of hydrogen atom, C₁₋₁₈ alkyl and C₂₋₁₈ alkenyl,
each of R₁ and R₂ is independently selected from the group consisting of substituted or unsubstituted C₃₋₂₀ cycloalkyl, substituted or unsubstituted C₃₋₂₀ cycloalkenyl, substituted or unsubstituted C₆₋₂₀ aryl, substituted or unsubstituted C₃₋₂₀ heterocycloalkyl, substituted or unsubstituted C₃₋₂₀ heterocycloalkenyl, and substituted or unsubstituted C₃₋₂₀ heteroaryl,
R₃ is selected from the group consisting of hydrogen atom, substituted or unsubstituted C₁₋₆ alkyl, substituted or unsubstituted C₃₋₆ carbocyclic group, -(CH₂)ₙQ, - (CH₂)ₙCHQR, -CHQR and -CQ(R)₂, wherein each R is independently selected from the group consisting of hydrogen atom, C₁₋₃ alkyl and C₂₋₃ alkenyl; Q is selected from the group consisting of carbocyclic group, heterocyclic group, -OR, -O(CH₂)ₙN(R)₂, -C(O)OR, -OC(O)R, -CX₃, -CX₂H, -CXH₂, -CN, -N(R)₂, -C(O)N(R)₂, -N(R)C(O)R, -N(R)S(O)₂R, -N(R)C(O)N(R)₂, -N(R)C(S)N(R)₂, -N(R)R₁₂, N(R)S(O)2R₁₂, -O(CH2)ₙOR, -N(R)C(=NR₁₃)N(R)₂, -N(R)C(=CHR₁₃)N(R)₂, -OC(O)N(R)₂, - N(R)C(O)OR, -N(OR)C(O)R, -N(OR)S(O)₂R, -N(OR)C(O)OR, -N(OR)C(O)N(R)₂, - N(OR)C(S)N(R)₂, -N(OR)C(=NR₁₃)N(R)₂, -N(OR)C(=CHR₁₃)N(R)₂, -C(=NR₁₃)N(R)₂, - C(=NR₁₃)R, -C(O)N(R)OR and -C(R)N(R)₂C(O)OR, wherein each n is independently an integer of from 1 to 5; R₁₂ is selected from the group consisting of C₃₋₆ carbocyclic group and heterocyclic group; R₁₃ is selected from the group consisting of H, CN, NO₂, C₁₋₆ alkyl, -OR, -S(O)₂R, - S(O)₂N(R)₂, C₂₋₆ alkenyl, C₃₋₆ carbocyclic group and heterocyclic group; each R is independently selected from the group consisting of hydrogen atom, C₁₋₃ alkyl and C₂₋₃ alkenyl; each X is independently selected from the group consisting of F, CI, Br and I, provided that when R₃ is - (CH₂)ₙQ, -(CH₂)ₙCHQR, -CHQR or -CQ(R)₂, (i) if n is 1, 2, 3, 4, or 5, then Q is not -N(R)₂, or (ii) if n is 1 or 2, Q is not 5-, 6- or 7-membered heterocycloalkyl,
each of R₄ to R₁₁ is independently selected from the group consisting of hydrogen atom, C₁₋₃ alkyl and C₂₋₃ alkenyl, and
each of a, b, c and d is independently an integer of from 1 to 15.

3. The composition for drug delivery according to claim 1, wherein
each of M₁ and M₂ is independently selected from the group consisting of -C(O)O-, - OC(O)-, -OC(O)-M'-C(O)O-, -C(O)N(R')-, -N(R')C(O)- and -C(O)-, wherein M' and R' are the same as defined in Claim 2,
each of R₁ and R₂ is independently selected from the group consisting of substituted or unsubstituted C₃₋₂₀ cycloalkyl and substituted or unsubstituted C₃₋₂₀ heterocycloalkyl,
R₃ is selected from the group consisting of hydrogen atom, substituted or unsubstituted C₁₋₆ alkyl, and substituted or unsubstituted C₃₋₆ carbocyclic group,
each of R₄ to R₁₁ is independently hydrogen atom or C₁₋₃ alkyl, and
each of a, b, c and d is independently an integer of from 3 to 11.

4. The composition for drug delivery according to claim 1, wherein
each of M₁ and M₂ is independently -C(O)O- or -OC(O)-,
each of R₁ and R₂ is independently substituted or unsubstituted C₃₋₆ cycloalkyl,
R₃ is hydrogen atom or unsubstituted C₁₋₃ alkyl,
R₄ to R₁₁ are hydrogen atom, and
each of a, b, c and d is independently an integer of from 5 to 9.

5. The composition for drug delivery according to claim 1, wherein the lipid is one having a structure selected from the following formulas A to O:
| Formula | Structure |
|---|---|
| A | 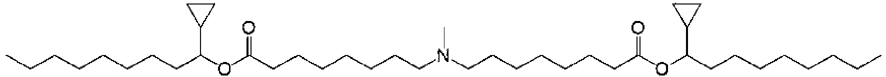 |
| B | 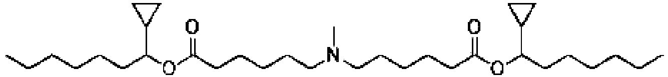 |
| C | 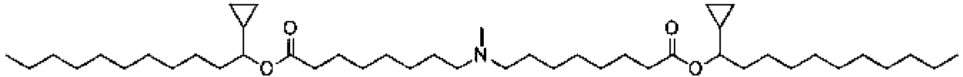 |
| D | 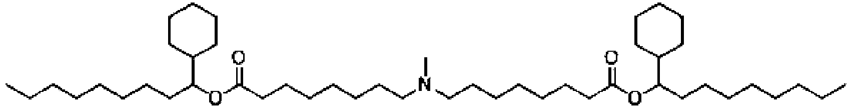 |
| E | 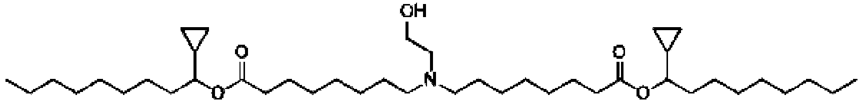 |
| F | 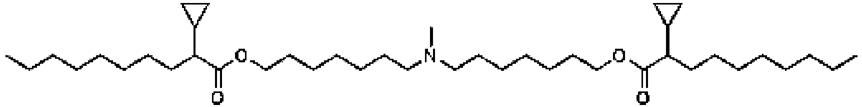 |
| G | 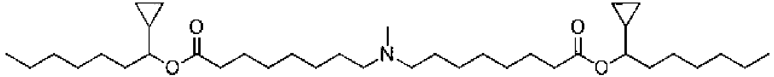 |
| H | 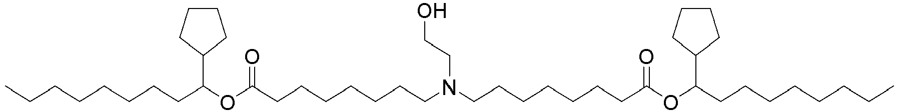 |
| I | 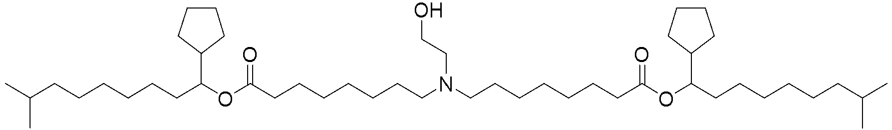 |
| J | 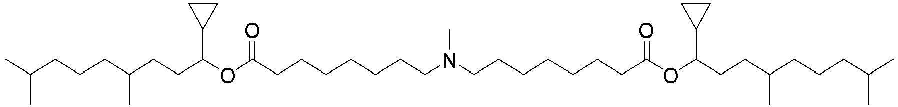 |
| K | 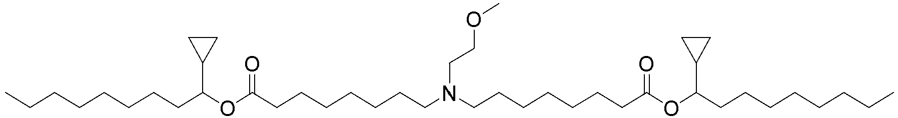 |
| L | 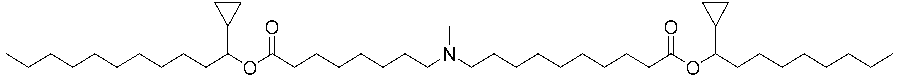 |
| M | 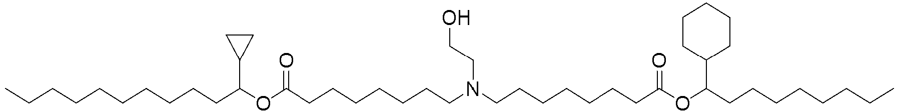 |
| N | 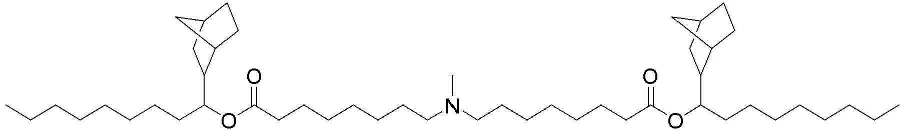 |
| O | 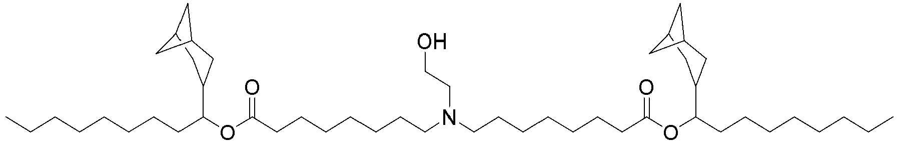 |

6. The composition for drug delivery according to claim 1, wherein the effective ingredient is mRNA.

7. The composition for drug delivery according to claim 1, wherein the lipid-polymer is a polymer in which one or more saturated or unsaturated hydrocarbon groups having 11 to 25 carbon atoms as a hydrophobic part are introduced into a hydrophilic block which is a hydrophilic part.

8. The composition for drug delivery according to claim 7, wherein the hydrophilic block is one or more selected from the group consisting of polyalkylene glycol, polyvinyl alcohol, polyvinyl pyrrolidone, polyacrylamide, and derivatives thereof.

9. The composition for drug delivery according to claim 7, wherein the saturated or unsaturated hydrocarbon group having 11 to 25 carbon atoms is independently selected from the group consisting of myristoyl, dimyristoyl, lauryl, myristyl, palmityl, stearyl, arachidyl, behenyl, lignoceryl, cerotyl, myristoleyl, palmitoleyl, sapienyl, oleyl, linoleyl, arachidonyl, eicosapentaenyl, erucyl, and docosahexaenyl.

10. The composition for drug delivery according to claim 1, wherein the amphiphilic block copolymer is an A-B type block copolymer comprising a hydrophilic A block and a hydrophobic B block, wherein the hydrophilic A block is one or more selected from the group consisting of polyalkylene glycol, polyvinyl alcohol, polyvinyl pyrrolidone, polyacrylamide, and derivatives thereof, and the hydrophobic B block is one or more selected from the group consisting of polyester, polyanhydride, polyamino acid, polyorthoester and polyphosphazine.

11. The composition for drug delivery according to claim 10, wherein the hydroxyl group at the end of the hydrophobic B block is modified with one or more selected from the group consisting of cholesterol, tocopherol, and fatty acids having 10 to 24 carbons.

12. The composition for drug delivery according to any one of claims 1 to 11, wherein the composition further comprises fusogenic lipid.

13. The composition for drug delivery according to claim 12, wherein the fusogenic lipid is one or a combination of two or more selected from the group consisting of phospholipid, cholesterol, and tocopherol.

14. A method for preparing a composition for drug delivery, comprising the steps of:
(a) preparing a solution in which a lipid represented by the following formula 1; and lipid-polymer, amphiphilic block copolymer, or a mixture thereof; are dissolved in a water-miscible organic solvent; and
(b) to the solution prepared in step (a), adding effective ingredient selected from nucleic acid, polypeptide, virus, or combination thereof, and mixing them: 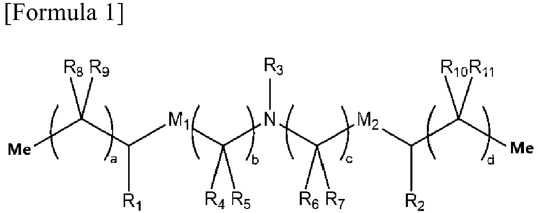 wherein, in the above formula 1,
each of M₁ and M₂ is independently a divalent linker group,
each of R₁ and R₂ is independently a substituted or unsubstituted carbocyclic group or heterocyclic group,
R₃ is hydrogen atom, or a substituted or unsubstituted organic group optionally comprising one or more heteroatoms,
each of R₄ to R₁₁ is independently hydrogen atom, or a substituted or unsubstituted, saturated or unsaturated hydrocarbon group,
Me is methyl group, and
each of a, b, c and d is independently an integer of from 1 to 20.

15. The method for preparing a composition for drug delivery according to claim 14, wherein the water-miscible organic solvent in step (a) is ethanol.

16. The method for preparing a composition for drug delivery according to claim 14, wherein step (b) comprises:
(b-1) a step of preparing a buffer solution containing the effective ingredient; and
(b-2) a step of adding the buffer solution of the effective ingredient prepared in step (b-1) to the solution prepared in step (a), and mixing them.

17. The method for preparing a composition for drug delivery according to claim 14, wherein step (b) comprises:
(b-1) a step of adding the effective ingredient to the solution prepared in step (a); and
(b-2) a step of adding a buffer solution to the resulting mixture of step (b-1) and mixing them.
